# EUROPEAN PATENT SPECIFICATION

(11) **EP 2 875 147 B1**
(45) Date of publication and mention of the grant of the patent: **05.07.2017**
(21) Application number: 13823685.6
(22) Date of filing: 18.07.2013
(51) Int. Cl.: C12Q 1/48, C12Q 1/68, C12N 9/10

(54) **DUAL ENZYMATIC AMPLIFICATION**
DUALE ENZYMATISCHE AMPLIFIKATION
AMPLIFICATION ENZYMATIQUE DOUBLE

(30) Priority: 23.07.2012 US 201261674696 P
(43) Date of publication of application: 27.05.2015
(73) Proprietor: Cynvenio Biosystems, Inc., Westlake, CA 91361 (US)
(72) Inventor: STRAUSS, William M., Westlake Village, California 91362 (US)
(74) Representative: Browne, Robin Forsythe
(86) International application number: PCT/US2013/051081
(87) International publication number: WO 2014/018367

(56) References cited:
- US-A1- 2003 049 635
- US-A1- 2003 049 635
- US-A1- 2003 180 741
- US-A1- 2011 262 898
- William M Strauss ET AL: "Rare Cell Analysis Without Whole Genome Amplification Rare Cell Analysis Without Whole Genome Amplification By Massively Parallel Sequencing", , 30 July 2012 (2012-07-30), pages 1-12, XP055247168, Retrieved from the Internet: URL:https://static1.squarespace.com/static /526eafafe4b0c46729f78d96/t/5283c39ce4b05f 14e259483b/1384367004839/Strauss-Genomics Studies Using Massively Parallel Sequencing.pdf [retrieved on 2016-02-03]
- I. KINDE ET AL: "Detection and quantification of rare mutations with massively parallel sequencing", PROCEEDINGS OF THE NATIONAL ACADEMY OF SCIENCES, vol. 108, no. 23, 17 May 2011 (2011-05-17) , pages 9530-9535, XP055164202, ISSN: 0027-8424, DOI: 10.1073/pnas.1105422108
- JOE SHUGA ET AL.: 'Selected Technologies for Measuring Acquired Genetic Damage in Humans.' ENVIRONMENTAL AND MOLECULAR MUTAGENESIS vol. 11, 2010, pages 851 - 870, XP055188549
- AREE THATTIYAPHONG.: 'Mimetic peptides to o-polysaccharide antigens of Vibrio Cholerae O1 and 0139.' A THESIS SUBMITTED IN PARTIAL FULFILLMENT OF THE REQUIREMENTS FOR THE DEGREE OF DOCTOR OF PHYLOSOPHY (BIOSCIENCE) GRADUATE SCHOOL 2007, pages 28 - 30, XP055188554
- HISHAM MOHAMED ET AL.: 'Isolation of tumor cells using size and deformation' JOURNAL OF CHROMATOGRAPHY A vol. 1216, 2009, pages 8289 - 8295, XP026721730

## Description

### FIELD OF THE INVENTION

The present invention relates to methods for validating the presence and character of genomic mutations, particularly single nucleotide polymorphisms (SNPs), by parallel amplification of a portion or the whole genome with at least two different DNA polymerases.

### BACKGROUND OF THE INVENTION

Solid tissue cancers start to grow at a primary site. As the disease progresses, metastases arise at distant locations. These metastatic events accelerate the disease and eventually lead to death. Cells or fragments of cells leave the primary site as part of the metastatic process. The process of metastasis is complex. Part of the metastatic process involves rare circulating tumor cells (CTC). That these CTC are not a monolithic population within a given patient is becoming clear. Fractionation of the CTC's within a patient is essential to understand the mutations responsible for the cancer afflicting the patient. Purification and isolation of these rare tumor cells (or cell derived events) is required to define oncogenic mutations in patient blood. Many cells and cell fragments exist in whole blood that do not contain mutations, thus in order to isolate the useful mutation bearing cells, a purification strategy is required.

In order to measure mutations in the DNA genome of CTC's isolated from small volumes of whole blood by any technology, DNA of sufficient quantity and quality is important. Typically, in 2 to 4 ml of whole blood one can expect in the range of about 2 to 10 CTCs to be recovered. This number of cells must be processed with excellent recovery to ensure that mutation-bearing chromosomes are not lost during processing. Thus, to isolate DNA of sufficient quality and quantity a special approach is required. Conventional methods are not useful as they alter the DNA genomic representation, produce inferior quality DNA and/or result in insufficient quantity from such rare samples for use in a variety of molecular assays such as, but not limited to, quantitative PCR (QPCR) and DNA sequencing.

Use of whole genome amplified DNA has proven useful for the purpose of analyzing mutations in clinical samples so produced. However, following standard sequencing library protocols, using this DNA may produce inconsistent and/or inaccurate sequencing results.

Next Gen sequence technology, or any method of mutation detection relies upon the homogeneity and sufficient quantity of sample material to provide the assay with sampling significance. Cynvenio produces a device for rare cell isolation. Typical of these samples (produced by Cynvenio CTC isolation technology) only a few CTC cells can be recovered for each ml of whole blood. For very small samples, where only a few cells are provided, the standard template requirements for assay measurement cannot be met. We have used whole genome amplification to increase the amount of template to circumvent this limitation. However, whole genome amplification introduces errors into the sample which can prevent interpretable results.

### SUMMARY OF THE INVENTION

In one aspect, the invention provides methods for verifying the presence of a genomic mutation in cells of a rare cell population. In some embodiments, the methods comprise:
a) amplifying a portion or the whole genome of the cells of the rare cell population with a first DNA polymerase;
b) amplifying a portion or the whole genome of the cells of the rare cell population with a second DNA polymerase, wherein the second DNA polymerase is different from the first DNA polymerase, wherein the amplifying with the first DNA polymerase and second DNA polymerase are performed as parallel amplification reactions in separate reaction mixtures;
c) comparing the amplified genomic sequences obtained in steps a) and b) with an unamplified genomic sequence obtained from a control population of cells comprising normal somatic genomic DNA, wherein identification of a nucleotide polymorphism that is identical in the genomic sequences obtained in steps a) and b), but different from a nucleotide polymorphism at the same nucleotide position in the genomic sequence obtained the unamplified genomic sequence verify the presence of a genomic mutation in cells of the rare cell population. In various embodiments, the amplified and unamplified genomic sequences are compared by one or more procedures comprising sequencing, amplification and/or hybridization. In some embodiments, the presence or absence of the genomic mutation is detected by PCR. In some embodiments, the presence or absence of the genomic mutation is detected by microarray. In some embodiments, the presence or absence of the genomic mutation is detected by sequencing.

In another aspect, the invention provides methods for verifying the presence of a genomic mutation in cells of a rare cell population. In some embodiments, the methods comprise:
a) amplifying and sequencing a portion or the whole genome of the cells of the rare cell population with a first DNA polymerase;
b) amplifying and sequencing a portion or the whole genome of the cells of the rare cell population with a second DNA polymerase, wherein the second DNA polymerase is different from the first DNA wherein the amplifying with the first DNA polymerase and second DNA polymerase are performed as parallel amplification reactions in separate reaction mixtures;
c) sequencing without amplifying a portion or the whole genome of a control cell population comprising normal somatic genomic DNA;
d) comparing the genomic sequences obtained in steps a), b) and c), wherein identification of a nucleotide polymorphism that is identical in the genomic sequences obtained in steps a) and b), but different from a nucleotide polymorphism at the same nucleotide position in the genomic sequence obtained in step c) verify the presence of a genomic mutation in cells of the rare cell population.

In various embodiments, the first DNA polymerase and the second DNA polymerase have different error correction rates. In some embodiments, the first DNA polymerase and the second DNA polymerase have different nucleic acid copying fidelities. In some embodiments, the first DNA polymerase and/or the second DNA polymerase have 5' → 3' exonuclease activity. In some embodiments, the first DNA polymerase and/or the second DNA polymerase do not have 3' → 5' exonuclease activity. In some embodiments, the first DNA polymerase and/or the second DNA polymerase have helicase and/or strand displacement activity. In some embodiments, the first DNA polymerase and the second DNA polymerase are selected from the group consisting of a Φ29 (Phi29) DNA polymerase, a *Thermus aquaticus* (Taq) DNA polymerase, a *Thermus flavus* (Tfl) DNA polymerase, a *Thermus thermophilus* (rTth) DNA polymerase, a *Thermus clitoris* (Tli) DNA polymerase, a *Thermotoga maritima* (Tma) DNA polymerase, a *Pyrococcus furiosus* (Pfu) DNA polymerase, a *Bacillus stearothermophilus* (Bst) DNA polymerase, PHUSION® High-Fidelity DNA Polymerase, Vent_{R}^{®} DNA polymerase, Deep Vent_{R}™ DNA polymerase, a Q5™ High-Fidelity DNA polymerase, and REPLI-g DNA polymerase. In some embodiments, the first DNA polymerase and the second DNA polymerase are selected from the group consisting of a Φ29 DNA polymerase, a *Thermus aquaticus* (Taq) DNA polymerase, a *Thermus thermophilus* (rTth) DNA polymerase, a *Pyrococcus furiosus* (Pfu) DNA polymerase, a *Bacillus stearothermophilus* (Bst) DNA polymerase, and a PHUSION^{®} High-Fidelity DNA Polymerase. In some embodiments, the first DNA polymerase is a Φ29 DNA polymerase and the second DNA polymerase is a *Thermus aquaticus* (Taq) DNA polymerase. In some embodiments, the first DNA polymerase is a Φ29 DNA polymerase and the second DNA polymerase is a PHUSION^{®} High-Fidelity DNA polymerase.

In various embodiments, the methods further comprise the step detecting the presence, absence or character of one or more genomic mutations (*e*.*g*., SNPs) in the amplified and unamplified nucleic acid sequences. In various embodiments, the methods further comprise the step of isolating the genomic DNA from the cells of a rare cell population. In various embodiments, the methods further comprise the step of isolating the cells of the rare cell population. In various embodiments, the methods further comprise the step of obtaining the cells of the rare cell population from a subject. In various embodiments, the rare cell population is circulating tumor cells (CTC). In some embodiments, the CTC are obtained from a blood sample of a subject. In some embodiments, the CTC are isolated based on their surface expression of Epithelial cell adhesion molecule (Ep-CAM). In some embodiments, the CTC are isolated based on their expression of one or more CTC-associated markers, *e*.*g*., Epithelial cell adhesion molecule (Ep-CAM), keratin 19 (KRT19), mucin 1 (MUC1), carcinoembryonic antigen-related cell adhesion molecule 5 (CEACAM5), baculoviral IAP repeat containing 5 (BIRC5), secretoglobin, family 2A, member 2 (SCGB2A2), ERBB2, cytokeratin 8 (CK8), cytokeratin 18 (CK18) and cytokeratin 19 (CK19).

In some embodiments, the genomic mutation is a single nucleotide polymorphism (SNP).

In various embodiments, the somatic genomic DNA is from white blood cells (WBC). In various embodiments, the somatic genomic DNA is from a buccal swab. In various embodiments, the somatic genomic DNA is from a hair bulb or a hair follicle.

In some embodiments, the whole genome of the cells in steps a) and b) is amplified and sequenced. In some embodiments, a portion of the whole genome of the cells in steps a) and b) is amplified and sequenced. In some embodiments, the portion or the whole genome of the cells is sequenced by performing Next Generation Sequencing.

Also disclosed are methods for verifying the presence of a genomic mutation in cells of a rare cell population. In some embodiments, the methods comprise:
a) amplifying a portion or the whole genome of the cells of the rare cell population two or more iterations with a first DNA polymerase;
b) comparing the genomic sequences obtained in step a) with an unamplified genomic sequence obtained from a control population of cells comprising normal somatic genomic DNA, wherein identification of a nucleotide polymorphism that is identical in the genomic sequences obtained in step a), but different from a nucleotide polymorphism at the same nucleotide position in the genomic sequence obtained the unamplified genomic sequence verify the presence of a genomic mutation in cells of the rare cell population. In various embodiments, the amplified and unamplified genomic sequences are compared by one or more procedures comprising sequencing, amplification and/or hybridization. In some embodiments, the presence or absence of the genomic mutation is detected by PCR. In some embodiments, the presence or absence of the genomic mutation is detected by microarray. In some embodiments, the presence or absence of the genomic mutation is detected by sequencing.

Also disclosed are methods for verifying the presence of a genomic mutation in cells of a rare cell population. In some embodiments, the methods comprise:
a) amplifying and sequencing a portion or the whole genome of the cells of the rare cell population two or more iterations with a first DNA polymerase;
b) sequencing without amplifying a portion or the whole genome of a control cell population comprising normal somatic genomic DNA;
c) comparing the genomic sequences obtained in steps a) and b) with an unamplified genomic sequence obtained in step c), wherein identification of a nucleotide polymorphism that is identical in the genomic sequences obtained in step a), but different from a nucleotide polymorphism at the same nucleotide position in the genomic sequence obtained in step b) verify the presence of a genomic mutation in cells of the rare cell population.

In some embodiments, the first DNA polymerase has 5' → 3' exonuclease activity. In some embodiments, the first DNA polymerase does not have 3' → 5' exonuclease activity. In some embodiments, the first DNA polymerase has helicase and/or strand displacement activity. In some embodiments, the first DNA polymerase is selected from the group consisting of a Φ29 DNA polymerase, a *Thermus aquaticus* (Taq) DNA polymerase, a *Thermus flavus* (Tfl) DNA polymerase, a *Thermus thermophilus* (rTth) DNA polymerase, a *Thermus litoris* (Tli) DNA polymerase, a *Thermotoga maritima* (Tma) DNA polymerase, a *Pyrococcusfuriosus* (Pfu) DNA polymerase, a *Bacillus stearothermophilus* (Bst) DNA polymerase, PHUSION^{®} High-Fidelity DNA polymerase, VentR® DNA polymerase, Deep VentR™ DNA polymerase, a Q5™ High-Fidelity DNA polymerase, and REPLI-g DNA polymerase. In various embodiments, the first DNA polymerase is selected from the group consisting of a Φ29 DNA polymerase, a *Thermus aquaticus* (Taq) DNA polymerase, a *Thermus thermophilus* (rTth) DNA polymerase, a *Pyrococcus furiosus* (Pfu) DNA polymerase, a *Bacillus stearothermophilus* (Bst) DNA polymerase, and a PHUSION® High-Fidelity DNA polymerase.

In some embodiments, the methods further comprise the step of isolating the genomic DNA from the cells of a rare cell population. In some embodiments, the methods further comprise the step of isolating the cells of the rare cell population. In some embodiments, the methods further comprise the step of obtaining the cells of the rare cell population from a subject. In some embodiments, the rare cell population is circulating tumor cells (CTC). In some embodiments, the CTC are obtained from a blood sample of a subject. In some embodiments, the CTC are isolated based on their surface expression of Epithelial cell adhesion molecule (Ep-CAM). In some embodiments, the CTC are isolated based on their expression of one or more CTC-associated markers, e.g., Epithelial cell adhesion molecule (Ep-CAM), keratin 19 (KRT19), mucin 1 (MUC1), carcinoembryonic antigen-related cell adhesion molecule 5 (CEACAM5), baculoviral IAP repeat containing 5 (BIRC5), secretoglobin, family 2A, member 2 (SCGB2A2), ERBB2, cytokeratin 8 (CK8), cytokeratin 18 (CK18) and cytokeratin 19 (CK19).

In some embodiments, the genomic mutation is a single nucleotide polymorphism (SNP).

In some embodiments, the control cell population is from a cell population comprising normal somatic genomic DNA. In some embodiments, the somatic genomic DNA is from white blood cells (WBC). In various embodiments, the somatic genomic DNA is from a buccal swab. In various embodiments, the somatic genomic DNA is from a hair bulb or a hair follicle.

In some embodiments, the whole genome of the cells in steps a) and b) is amplified and sequenced. In some embodiments, a portion of the whole genome of the cells in steps a) and b) is amplified and sequenced. In some embodiments, the portion or the whole genome of the cells is sequenced by performing Next Generation Sequencing.

### DEFINITIONS

The term "rare cell population" refers to a cell population in a sample that is fewer than 1/10⁶ (*i.e.,* one in one million or 10⁻⁴ %) of the total cells in the sample, oftentimes fewer than 1/10⁷ (*i.e.,* one in ten million or 10⁻⁵ %), or fewer than 1/10⁸ (*i.e.,* one in one hundred million or 10⁻⁶ %), or fewer than 1/10⁹ *(i.e.,* one in one billion or 10⁻⁷%). An illustrative example of a rare cell population is circulating tumor cells (CTC). Circulating tumor cells are found in frequencies in the order of 1-10 CTC per mL of whole blood in patients with metastatic disease. For comparison, one milliliter of blood contains a few million white blood cells and a billion red blood cells.

Biological and biochemical terminology: Where specific categories of molecules are discussed, such as nucleic acids or proteins, synthetic forms are included, such as mimetic or isomeric forms of naturally occurring molecules. Unless otherwise indicated, modified versions are similarly encompassed, so long as the desired functional property is maintained. For example, an aptamer selective for a CD34 cell surface protein includes chemical derivatives (*e*.*g*., pegylated, creation of a pro-form, derivatized with additional active moieties, such as enzymes, ribozymes, etc.)

The term "biological fluid" denotes the source of the fluid, and includes (but is not limited to) amniotic fluid, aqueous humor, blood and blood plasma (and herein blood refers to the plasma component, unless otherwise expressly stated or indicated in context), cerumen (ear wax), Cowper's fluid, chime, interstitial fluid, lymph fluids, mammalian milk, mucus, pleural fluid, pus, saliva, sebum, semen, serum, sweat tears, urine, vaginal secretion, vomit and exudates (from wounds or lesions).

The terms "subject" or "individual" or "patient" refer to any mammal, for example a human or a non-human primate, a domesticated mammal (*e*.*g*., canine or feline), an agricultural mammal (*e.g.,* bovine, ovine, porcine, equine) or a laboratory mammal (*e.g.,* rat, mouse, rabbit, hamster, guinea pig).

The term "selective binding molecule" denotes a molecule that selectively, but not necessarily specifically, binds to a particular target moiety. The binding is not random. Selective binding molecules may be selected from among various antibodies or permutations (poly- or monoclonal, peptibodies, humanized, foreshortened, mimetics, and others available in the art), aptamers (which may be DNA, RNA, or various protein forms, and may be further modified with additional functional moieties, such as enzymatic or colorimetric moieties), or may be particular to a particular biological system. Proteins may be expressed with particular "tags" such as a "His-tag", and a skilled practitioner will determine appropriate kinds of selective binding molecules or detectable labels are suitable. The list is not exhaustive.

### BRIEF DESCRIPTION OF THE DRAWINGS

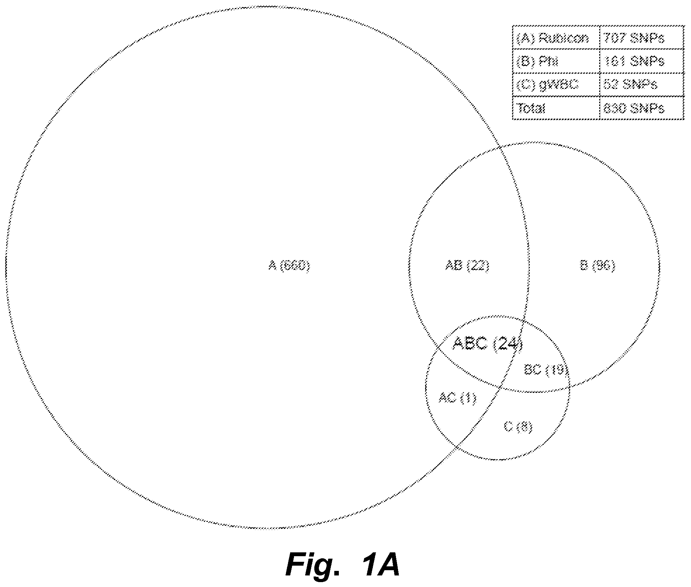

Figures 1A-D illustrates Boolean Analysis of libraries. In panel 1a gWBC (52 SNPs) is compared to gWBC+T(56 SNPs) and gWBC ∪ gWBC+T = 68. gWBC ∩ gWBC+T = 40 SNPs. By contrast in panel 1b Rubicon library (707 SNPs) is compared to Phi29 (161 SNPs) and to gWBC (52 SNPs) library. Rubicon ∪ Phi29 ∪ gWBC =830 SNPs , and Rubicon ∩ Phi29 ∩ gWBC =24 SNPs. In panel 1c, When three independent Phi29 libraries are compared such that Phi29A ∪ Phi29B ∪ Phi29C = 137 SNP, Phi29A ∩ Phi29B ∩ Phi29C = 32. Finally in panel 1d, three independent Rubicon libraries are compared: RubiconA ∪ RubiconB ∪ RubiconC = 707 SNPs, and RubiconA ∩ RubiconB ∩ RubiconC = 37 SNPs.

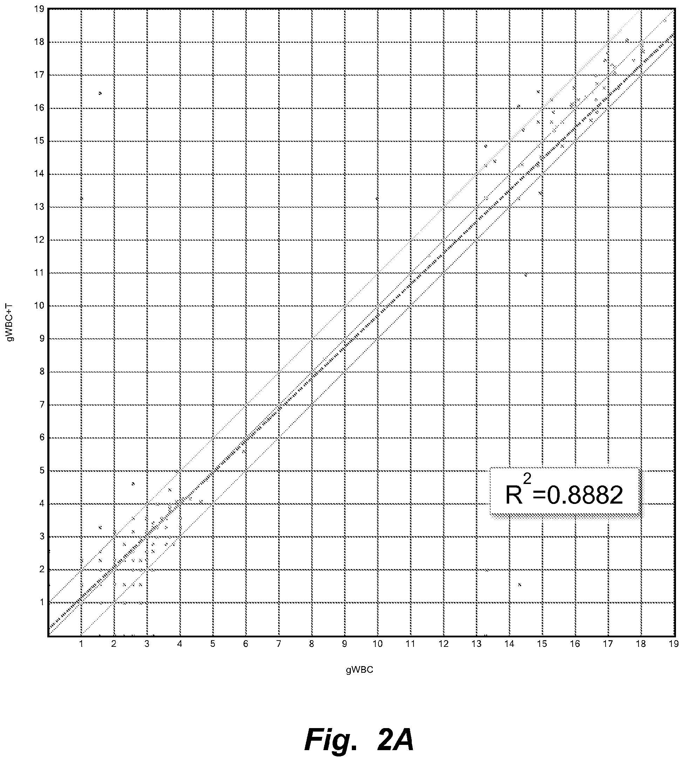

Figures 2A-F illustrates Scatter plots. One feature of the DNAStar ArrayStar package is a SNP Scatter plot calculator. This program assigns every SNP to a gene and gives it a numerical value depending upon the genomic consequences of the mutation. In SNP workflows, the Scatter Plot gives a visual comparison of gene level variation between any two samples. Each data point on the Scatter Plot represents an individual gene and the "signal" for gene is the sum of the weighted values for each class of variation: each synonymous SNP adds 1 to the signal, each non-synonymous SNP adds 100 and each nonsense or frameshift causing SNP adds 10,000. Values are halved where the change is heterozygous. These values can then be compared in a scatter plot graph. Panels 2a - 2d. Panel 2a, Scatter analysis between gWBC+T and Phi29 yields R2 = 0.7953. Panel 2b, Scatter analysis between gWBC+T and Rubicon yields R2 = 0.7025.

### DETAILED DESCRIPTION

### 1. Introduction

The present invention is based, in part, on the discovery of enzymatic amplification methods to control for artifacts, and to validate mutation data from small samples of tumor cells derived from patient samples, *e*.*g*., blood samples. In various embodiments, the methods find use in mutation detection in cancer patient samples, and circulating tumor cell identification and characterization.

In order to differentiate valid mutations from artifacts associated with the small amount of tumor sample, one must control for artifacts associated with the genomic amplification and sequencing methodology. To control for these artifacts a method is required that compares normal somatic DNA to CTC samples amplified by different enzymatic means. The present invention is based, in part, on the use of at least two different enzymatic methods in paired samples to create at least two amplified libraries from the same starting materials but by at least two different enzymatic reactions. These library comparisons, made by aligning sequence results from each of the different samples, will control for artifacts of amplification as each amplification reaction uses a different enzymology. Genomic mutations, or SNPs, found in both CTC sample sets but not in the control somatic DNA are considered to be real or validated mutations.

The method utilizes an unamplified, directly sequenced control sample, e.g., from a portion of recovered CTCs if available or from a genomic sample from healthy tissue, for example, white blood cell (WBC) pellet and at least two portions of CTC samples. If there are sufficient CTC per sample then a third sample is preferred. The control sample comprising somatic genomic DNA *(e.g.,* WBC, buccal swab, hair follicle) sample is not amplified but the genomic DNA is isolated and used to construct the first sequencing library. Two CTC samples are used to make two different amplified sample using two different amplification protocols. In one embodiment, one library from Phi29 polymerase based WGA technology (GE Healthcare, GenomePHL isothermal reaction) and the other library using a thermostable WGA protocol like the RUBICON technology using polymerase like PHUSION^{®} or Taq and random annealing primers were produced. Thus at a minimum, there were two different whole genome amplified (WGA) templates from CTC and one non-amplified template, in this case, not containing CTC *(e.g.,* normal somatic genomic material). In situations where a high percentage of CTCs are in the purified CTC sample (*e*.*g*., greater than 50%) then another purified CTC sample can be used to make a non-amplified CTC sample template. All sample templates, both control non-amplified and amplified are then sequenced. After sequencing, the sequences are co-aligned using a sequence assembler that can perform multi-library assemblies (*e*.*g*., the DNA Star NGEN assembler).

After multi-library assembly, SNPs are compared. SNPs that are not found in the WBC sequence library, but are present in both different types of amplified libraries are considered to be real or validated SNPs. SNP sequences from non-amplified CTC template sequences can be used when available as a further control.

Any mutation found in the WBC sample library is scored as a negative, any mutation not found in the WBC and found in both of the amplified libraries is scored as a positive, disease associated mutation. If the unamplified CTC sample is available, a true, disease-associated mutation would be found in the two WGA CTC libraries and the one non-WGA CTC library.

### 2. Obtaining a Biological Sample Comprising a Rare Cell Population

In various embodiments, the methods further comprise the step of obtaining a biological sample suspected of comprising one or more cells of the rare cell population. The biological sample can comprise cultured cells or be obtained from a subject. In various embodiments, the biological sample from the subject is a fluid biological sample, *e.g.,* amniotic fluid, aqueous humor, blood and blood plasma (and herein blood refers to the plasma component, unless otherwise expressly stated or indicated in context), cerumen (ear wax), Cowper's fluid, chime, interstitial fluid, lymph fluids, mammalian milk, mucus, pleural fluid, pus, saliva, sebum, semen, serum, sweat tears, urine, vaginal secretion, vomit and exudates (from wounds or lesions).

In some embodiments, the biological sample is a whole blood sample from a subject. In cases where the rare cell population sought to be analyzed is CTC, the subject is suspected of having CTC in the biological sample, *e.g.,* in a whole blood sample.

### 3. Isolating the Rare Cell Population from the Sample

In various embodiments, the methods further comprise the step of isolating the rare cell population. The rare cell population can be isolated using any appropriate or applicable method known in the art. In some embodiments, the rare cell population can be isolated based on the surface expression of a marker. For example, a solid support attached to a cognate binding partner of the protein marker can be used to capture and isolate the cells of the rare cell population. In various embodiments, the solid support is a magnetic bead attached (*e*.*g*., conjugated or covalently bound) to a cognate binding partner of a marker. Such methods are well known in the art.

In embodiments where the rare cell population is a CTC, the CTC can be concentrated and/or isolated based on their expression of one or more known CTC-associated markers, *e*.*g*., Epithelial cell adhesion molecule (Ep-CAM), keratin 19 (KRT19), mucin 1 (MUC1), carcinoembryonic antigen-related cell adhesion molecule 5 (CEACAM5), baculoviral IAP repeat containing 5 (BIRC5), secretoglobin, family 2A, member 2 (SCGB2A2), ERBB2, cytokeratin 8 (CK8), cytokeratin 18 (CK18) and cytokeratin 19 (CK19). Cognate binding partners to such surface expressed markers (*e*.*g*., a cognate ligand or antibody that binds to the marker) can be attached to a solid support, *e*.*g*., magnetic beads, and used to concentrate and/or isolate the CTC. In various embodiments, the CTC in a biological sample are enriched by removing CD45+ leukocytes from cells in the sample.

Methods for concentrating and/or isolating CTC described in the art find use. Illustrative methods for concentrating and/or isolating CTC are taught, *e.g.,* in U.S. Patent Publication Nos. 2011/0137018; 2011/0127222; 2011/0003303; 2010/0317093; and 2009/0053799. Additional methods for concentrating and/or isolating CTC that can be applied to the present methods are described, *e.g.,* in Lin, et al., Biosens Bioelectron. (2012) Jun 28., PMID 22784495; Yang, et al., Technol Cancer Res Treat. (2012) Jul 10. PMID 22775338; O'Brien, et al., J Biomed Opt. (2012) Jun;17(6):061221, PMID 22734751; Hughes, et al., J Vis Exp. (2012) Jun 15;(64). pii: 4248. doi: 10.3791/4248, PMID 22733259; Kim, et al., Lab Chip. (2012) Jun 11, PMID 22684249; Yu, et al., J Cell Biol. 2011 Feb 7;192(3):373-82; and Danova, et al., Expert Rev Mol Diagn. (2011) Jun;11(5):473-85. Further applicable methods and systems of use in concentrating and/or isolating CTC are described, e.g., in U.S. Patent Publication Nos. 2012/0129252; 2012/0100560; 2012/0045828; and 2011/0059519.

### 4. Isolating Genomic DNA

In various embodiments, the methods further comprise the step of purifying and/or isolating genomic DNA. Methods for purifying and/or isolating genomic DNA are well known in the art and can be applied in the present methods. Commercially available kits for purifying and/or isolating genomic DNA are readily available for purchase.

Basic methodologies for purifying and/or isolating genomic DNA are described, *e.g.,* in Sambrook and Russell, Molecular Cloning: A Laboratory Manual, Third Edition, 2001, Cold Spring Harbor Laboratory Press; and Ausubel, et al., Current Protocols in Molecular Biology, Wiley, updated through July 2, 2012. Kits of use for purifying and/or isolating genomic DNA can be purchased from numerous sources, including *e*.*g*., QIAGEN (on the internet at qiagen.com); Promega (on the internet at promega.com); Life Technologies (on the internet at invitrogen.com); G Biosciences (on the internet at gbiosciences.com); Sigma-Aldrich (on the internet at sigmaaldrich.com); Affymetrix (on the internet at affymetrix.com); and Fermentas Molecular Biology Tools (on the internet at fermentas.com).

### 5. Amplifying Genomic DNA

The methods comprise amplifying a portion of the genomic DNA or the entirety of genomic DNA (whole genome amplification or WGA) in a sample. Amplification methods are known in the art and can be applied in the present methods. Methods for whole genome amplification are described, *e*.*g*., in "Whole Genome Amplification: Methods Express Series", Hughes and Lasken, eds., 2005, 1st Edition, Scion Publishing Ltd.. Kits for whole genome amplification can be purchased from numerous sources, including *e*.*g*., QIAGEN (on the internet at qiagen.com); Sigma-Aldrich (on the internet at sigmaaldrich.com); and Rubicon Genomics (on the internet at rubicongenomics.com). Generally, the methods employ a DNA polymerase suitable for amplifying a substantial portion or the whole genome of a cell. Such DNA polymerases may have one or more attributes selected from, *e.g.,* high processivity, high fidelity, helicase activity, and/or 5'-3'-exonuclease activity. When the methods perform amplifying a portion of the genome, the same portion of the genome is amplified for useful comparisons (*e*.*g*., using the same primers), whether amplifying using multiple DNA polymerases or the same DNA polymerase in multiple iterations of amplification.

### a. Amplifying Using First and Second DNA Polymerases

In various embodiments, a portion or the entire genome of at least one cell of the rare cell population is subject to parallel amplifications using multiple different DNA polymerases, *e*.*g*., at least a first DNA polymerase and a second DNA polymerase. In varying embodiments, the first DNA polymerase, the second DNA polymerase, and any additional DNA polymerases, will have different amplification capabilities/attributes, including, *e*.*g*., different error correction rates, different processivities, different nucleic acid copying fidelities, different amplification biases, different levels of helicase activity, different 5'-3'-exonuclease activity and/or different 3'-5'-exonuclease activity.

Depending on the pairing of the first and second (and subsequent) DNA polymerases, the parallel amplification reactions can be performed in the same or different reaction mixtures. In some embodiments, the parallel amplification reactions with the first DNA polymerase and second DNA polymerase are performed in a single reaction mixture (*i.e.,* single reaction tube). In some embodiments, the parallel amplification reactions with the first DNA polymerase and second DNA polymerase are performed in separate reaction mixtures *(i.e.,* separate reaction tubes). In embodiments where the parallel amplification reactions are performed in separate reaction mixtures, the genomic DNA source material is divided into separate portions for each reaction mixture. If there is sufficient genomic DNA source material, further portions may be reserved for unamplified control reactions.

In various embodiments, the first DNA polymerase and the second DNA polymerase have different error correction rates. In some embodiments, the first DNA polymerase and the second DNA polymerase have different nucleic acid copying fidelities. In some embodiments, the first DNA polymerase and/or the second DNA polymerase have 5 → 3' exonuclease activity. In some embodiments, the first DNA polymerase and/or the second DNA polymerase do not have 3' → 5' exonuclease activity. In some embodiments, the first DNA polymerase and/or the second DNA polymerase have helicase and/or strand displacement activity. In some embodiments, the first DNA polymerase and the second DNA polymerase are selected from the group consisting of a Φ29 (Phi29) DNA polymerase, a *Thermus aquaticus* (Taq) DNA polymerase, a *Thermus flavus* (Tfl) DNA polymerase, a *Thermus thermophilus* (rTth) DNA polymerase, a *Thermus litoris* (Tli) DNA polymerase, a *Thermotoga maritima* (Tma) DNA polymerase, a *Pyrococcus furiosus* (Pfu) DNA polymerase, a *Bacillus stearothermophilus* (Bst) DNA polymerase, PHUSION^{®} High-Fidelity DNA Polymerase, Vent_{R}^{®} DNA polymerase, Deep Vent_{R}™ DNA polymerase, a Q5™ High-Fidelity DNA polymerase, and REPLI-g DNA polymerase. In some embodiments, the first DNA polymerase and the second DNA polymerase are selected from the group consisting of a Φ29 DNA polymerase, a *Thermus aquaticus* (Taq) DNA polymerase, a *Thermus thermophilus* (rTth) DNA polymerase, a *Pyrococcus furiosus* (Pfu) DNA polymerase, a *Bacillus stearothermophilus* (Bst) DNA polymerase, and a PHUSION^{®} High-Fidelity DNA Polymerase. In some embodiments, the first DNA polymerase is a Φ29 DNA polymerase and the second DNA polymerase is a *Thermus aquaticus* (Taq) DNA polymerase. In some embodiments, the first DNA polymerase is a Φ29 DNA polymerase and the second DNA polymerase is a PHUSION^{®} High-Fidelity DNA polymerase.

### b. Multiple Amplifications Using First Polymerase

In various embodiments, a portion or the entire genome of at least one cell of the rare cell population is subject to multiple *(i.e.,* two or more iterations of) amplification reactions using the same DNA polymerase. For this embodiment, the genomic DNA source material is divided into a separation portion for each iteration of amplification reaction (e.g., for each amplification of a portion or the entire genome). Each iteration of amplification is performed in a separate reaction mixture, using the same DNA polymerase for each iteration. If there is sufficient genomic DNA source material, further portions may be reserved for unamplified control reactions.

In some embodiments, the first DNA polymerase has 5' → 3' exonuclease activity. In some embodiments, the first DNA polymerase does not have 3' → 5' exonuclease activity. In some embodiments, the first DNA polymerase has helicase and/or strand displacement activity. In some embodiments, the first DNA polymerase is selected from the group consisting of a Φ29 DNA polymerase, a *Thermus aquaticus* (Taq) DNA polymerase, a *Thermus flavus* (Tfl) DNA polymerase, a *Thermus thermophilus* (rTth) DNA polymerase, a *Thermus litoris* (Tli) DNA polymerase, a *Thermotoga maritima* (Tma) DNA polymerase, a *Pyrococcus furiosus* (Pfu) DNA polymerase, a *Bacillus stearothermophilus* (Bst) DNA polymerase, PHUSION^{®} High-Fidelity DNA polymerase, VentR® DNA polymerase, Deep VentR™ DNA polymerase, a Q5™ High-Fidelity DNA polymerase, and REPLI-g DNA polymerase. In various embodiments, the first DNA polymerase is selected from the group consisting of a Φ29 DNA polymerase, a *Thermus aquaticus* (Taq) DNA polymerase, a *Thermus thermophilus* (rTth) DNA polymerase, a *Pyrococcus furiosus* (Pfu) DNA polymerase, a *Bacillus stearothermophilus* (Bst) DNA polymerase, and a PHUSION® High-Fidelity DNA polymerase.

### 6. Detecting Genomic Mutations

In various embodiments, the methods comprise the step of detecting the presence, absence and/or character of one or more genomic mutations (*e.g.,* (*e.g.,* single nucleotide polymorphisms or SNPs) in the amplified and unamplified nucleic acid sequences. Various assays may be used to characterize genomic mutations (*e*.*g*., SNPs) in one or more genomic regions of interest. For example, suitable methods may involve enumerating individual nucleic acid molecules/fragments containing a genomic region of interest or measuring signal intensity changes for polymorphic probes (*e*.*g*., SNP specific probes) on a microarray (*e*.*g*., using array-based comparative genomic hybridization (aCGH) technology). Various methods may be used to enumerate individual nucleic acid molecules including, but not limited to, DNA sequencing (*e*.*g*., high throughput single molecule sequencing), digital PCR, bridge PCR, emulsion PCR, nanostring technology, among others. Exemplary methods are described in more detail below.

The presence or absence of genomic mutations (*e*.*g*., SNPs) are detected in the amplified test genomic DNA sequences from the rare cell population (*e*.*g*., CTC) sample as well in the unamplified normal control DNA comprising somatic genomic DNA.

### a. Single Molecule Sequencing

In various embodiments, an amplified portion or the whole genome are sequenced. In certain embodiments of the invention, methods comprise single molecule sequencing of nucleic acids in the sample, for example, in order to characterize and/or quantify a genomic region with certain sequence composition. In particular, single molecule sequencing techniques allow the evaluation of individual nucleic acid molecules with polymorphic nucleotides and obtaining sequence read counts attributable to distinct polymorphic regions.

Various single molecule sequencing methods have been described in the art and can be used to detect genomic mutations (*e.g.,* SNPs). *See, e.g.,* Braslaysky et al., (2003), Proc. Natl. Acad. Sci., 100: 3960-64; Greenleaf et al., (2006), Science, 313: 801; Harris et al., (2008) Science, 320:106-109; Eid et al., (2009), Science, 323:133-138; Pushkarev et al., (2009), Nature Biotechnology, 27:847-850. Typically, in single molecule sequencing techniques, nucleic acid fragments, which serve as templates during sequencing reactions, are immobilized to a solid support such that at least a portion of the nucleic acid fragment is individually optically-resolvable.

Solid supports suitable for the invention can be any solid surface to which nucleic acids can be covalently attached, such as, for example latex beads, dextran beads, polystyrene, polypropylene surface, polyacrylamide gel, gold surfaces, glass surfaces and silicon wafers. In some embodiments, solid support is a glass surface. In some embodiments, the solid support is a slide, *e.g.,* a glass slide.

Means for attaching nucleic acids to a solid support as used herein refers to any chemical or non-chemical attachment method including chemically-modifiable functional groups. "Attachment" relates to immobilization of nucleic acid on solid supports by either a covalent attachment or via irreversible passive adsorption or via affinity between molecules (for example, immobilization on an avidin-coated surface by biotinylated molecules). Typically, the attachment is of sufficient strength that it cannot be removed by washing with water or aqueous buffer under DNA-denaturing conditions. "Chemically-modifiable functional group" as used herein refers to a group such as, for example, a phosphate group, a carboxylic or aldehyde moiety, a thiol, or an amino group.

In some embodiments, a solid support suitable for the invention has a derivatised surface. In some embodiments, the derivatised surface of the solid support is subsequently modified with bifunctional crosslinking groups to provide a functionalized surface, preferably with reactive crosslinking groups. "Derivatised surface" as used herein refers to a surface which has been modified with chemically reactive groups, for example amino, thiol or acrylate groups. "Functionalized surface" as used herein refers to a derivatised surface which has been modified with specific functional groups, for example the maleic or succinic functional moieties.

In some embodiments, each molecule of a nucleic acid fragment (which may comprise all or part of a genomic region) is attached to the solid support at a distinct location. In some embodiments, nucleic acid fragments that are immobilized to a solid support are detectably labeled (e.g., labeled with a detectable moiety that can generate an optical signal). For example, the nucleic acid fragments may be annealed to an oligonucleotide primer that is detectably labeled. Locations of each single molecule on the solid support may be read by an instrument that detects the label (*e*.*g*., detectable moiety), and the locations of each molecule recorded. In some embodiments, the detectable label of the nucleic acid fragment is removed after locations are recorded. For example, in embodiments in which the detectable label comprises a fluorescent moiety, the detectable label may be removed by photobleaching the fluorescent moiety. Alternatively or additionally, the detectable label may be cleaved off of the nucleic acid fragment.

In some embodiments, capturing oligonucleotides are immobilized on the solid or semisolid support to facilitate capturing and immobilization of nucleic acid fragments (*e*.*g*., polynucleotides), as described further herein.

Sequencing reactions can be performed using the immobilized nucleic acid fragments as templates. Primers are hybridized to the nucleic acid fragments to form a primer/template duplex. In some embodiments, nucleic acid fragments are modified to include adapters that are complementary to primers used. In some embodiments, primers are immobilized onto solid surfaces and nucleic acid fragments are attached to solid surfaces via their hybridization with primers.

Methods for sequencing the entire genome or a substantial portion of a cell are known in the art and can be applied in the present methods. For example, methods for whole genome amplification are described, *e*.g., in "Whole Genome Sequencing," Parthalan (Editor), VadPress (2012), and reviewed in Ross, et al., Am J Clin Pathol. (2011) 136(4):527-39. In various embodiments, full genome sequencing can be accomplished by any technology known in the art, including, *e*.*g*., nanopore technology (offered through Illumina (on the internet at illumina.com)); fluorophore technology (offered through Pacific Biosciences (on the internet at pacificbiosciences.com)), DNA Nanoball (DNB) technology (offered through Complete Genomics (on the internet at completegenomics.com)), and/or Pyrosequencing (offered by 454 Life Sciences (on the internet at 454.com)). Companies with whole genome sequencing platforms and sequence analysis tools of use in the present methods include, *e*.*g*., Illumina, Knome (on the internet at knome.com), Sequenom (on the internet at sequenom.com), 454 Life Sciences, Pacific Biosciences, Complete Genomics, Qiagen (via acquisition of Intelligent Bio-Systems), and Helicos Biosciences (on the internet at helicosbio.com).

In various embodiments, the amplified portion or the whole genome are sequenced using high-throughput sequencing or Next Generation Sequencing (NGS) techniques. Numerous high-throughput sequencing methods are known in the art and may be applied in the present methods. For example, in various embodiments, the amplified portion or the whole genome are sequenced by employing a technique, platform or methodology selected from Massively Parallel Signature Sequencing (MPSS) and/or Solexa (fluorescent-label-based) sequencing (offered through Illumina (on the internet at illumina.com)); Polony sequencing and/or SOLiD sequencing and/or Ion semiconductor sequencing (offered through Life Technologies (on the internet at lifetechnologies.com)); parallelized pyrosequencing (offered through on the internet at 454.com and Roche Diagnostics); DNA nanoball sequencing (offered through Complete Genomics (on the internet at completegenomics.com)); HeliScope™ single molecule sequencing (offered through Helicos Biosciences (on the internet at helicosbio.com)); Single molecule SMRT™ sequencing (offered through Pacific Biosciences (on the internet at pacificbiosciences.com)); Single molecule real time (RNAP) sequencing; and/or Nanopore DNA sequencing.

In some embodiments, single molecule sequencing is performed in a high-throughput fashion, *e*.*g*., with many sequencing reactions being performed in parallel. For example, a high throughput single molecule sequencing assay suitable for the invention may characterize up to thousands, millions, or billions of molecules simultaneously. Parallel sequencing reactions need not be performed synchronously; asynchronous reactions can be performed and are compatible with methods of the invention.

In some embodiments, a large portion (*e*.*g*., more than 10%, 15%, 20%, 25%, 30%, 35%, 40%, 45%, 50%, 55%, 60%, 65%, 70%, 75%, 80%, 85%, 90%, 95%, 99%, or more than 99%) of the genome is sequenced. In some embodiments, at least one genomic region that is sequenced is covered on average at least 10 times (10 x genome equivalents), that is, there are on average 10 reads or more of a given genomic region. In some embodiments, coverage is at least 20x, at least 30x, at least 40x, at least sox, at least 60x, at least 70x, at least 80x, at least 90x, at least 100x, at least 110x, at least 120x, or more times. In some embodiments, coverage is 100 times (100 x genome equivalents) or more.

In some embodiments, an unbiased nucleic acid sequencing method is employed. That is, the representation of a particular sequence among all the sequencing reads reflects the representation of the corresponding nucleic acid in the sample. In some embodiments, unbiased nucleic acid sequencing is achieved at least in part by not amplifying the template nucleic acids before the sequencing reaction. In some embodiments, the template nucleic acid is also not amplified during the sequencing reaction. In some embodiments, unbiased DNA sequence uses bright fluorophores and laser excitation to detect pyrosequencing events from individual DNA molecules fixed to a surface, eliminating the need for amplification.

In some embodiments, pyrosequencing (*i*.*e*., sequencing by synthesis) is performed. Specifically, template-dependent primer extension is performed in the presence of one or more nucleotides or nucleotide analogs (*e*.*g*., dNTPs) and one or more nucleic acid polymerases, under suitable conditions to allow extension of the primer by at least one base. Typically, nucleotides incorporated during sequencing reactions are detectably labeled (*e.g.,* labeled with a detectable moiety that can generate an optical signal). Signal emanating from the label is detected and recorded; a particular signal may be associated with the identity of a particular nucleotide or nucleotide analog, thus revealing the identity of the corresponding complementary nucleotide on the template nucleic acid fragment. In some embodiments, detectable signals are removed and/or destroyed after a round of incorporation (*e*.*g*., as described herein), thus facilitating further extension and detection of labeled nucleotides or nucleotide analogs.

Sequencing can be optimized to achieve rapid and complete addition of the correct nucleotide to primers in primer/template complexes, while limiting the misincorporation of incorrect nucleotides. For example, dNTP concentrations may be lowered to reduce misincorporation of incorrect nucleotides into the primer. Kₘ values for incorrect dNTPs can be as much as 1000-fold higher than for correct nucleotides, indicating that a reduction in dNTP concentrations can reduce the rate of misincorporation of nucleotides. Thus, in some embodiments, the concentration of dNTPs in the sequencing reactions are approximately 5-20 µM.

In addition, relatively short reaction times can be used to reduce the probability of misincorporation. For example, for an incorporation rate approaching the maximum rate of about 400 nucleotides per second, a reaction time of approximately 25 milliseconds will be sufficient to ensure extension of 99.99% of primer strands.

Detectable moieties may be directly or indirectly incorporated into nucleotides, nucleotide analogs, polynucleotides, or other molecules as appropriate. Suitable detectable moieties include, among other things, fluorescent moieties and luminescent moieties. In some embodiments, a fluorescent moiety comprises a cyanine dye, *e*.*g*., cyanine-3 and/or cyanine 5. Examples of suitable detectable moieties are described further herein.

Suitable reagents (*e*.*g*., nucleotides and/or nucleotide analogs, nucleic acid polymerases, etc.), solid supports, apparatuses, and methods of sequence analysis are known and have been described in the art. See, *e.g.,* U.S. Pat. Nos. 7,169,560; 7,220,549; 7,276,720; 7,279,563; 7,282,337; 7,397,546; 7,424,371; 7,476,734; 7,482,120; 7,491,498; 7,501,245; 7,593,109; 7,635,562; 7,666,593; 7,678,894; and 7,753,095. Various commercially available kits such as True Single Molecule Sequencing (tSMS)™ (Helicos) may be used to practice the present invention.

### b. Digital PCR

In some embodiments, digital PCR is used to characterize and/or quantify polymorphic genomic regions. Typically, digital PCR involves amplifying a single DNA template from minimally diluted samples, therefore generating amplicons that are exclusively derived from one template and can be detected with different fluorophores to discriminate and count different polymorphic regions. Thus, digital PCR transforms the exponential, analog signals obtained from conventional PCR to linear, digital signals, allowing statistical analysis of the PCR product.

Digital PCR technology is well described in the art. See, Vogelstein B. and Kinzler K. W., (1999), Proc. Natl. Acad. Sci. USA, Vol. 96, pp 9236-9241; Pohl G. and Shih L. M., (2004), Expert. Rev. Mol. Diagn., 4(1), 41-47.

In some embodiments, DNA prepared from a sample is first diluted onto multi-well (*e.g.,* 96-well, 384-well) plates with one template per two wells on average (*i.e.,* 0.5 template molecules (genomic equivalent) per well on average). To determine optimal dilution, DNA can be first quantified to determine the amount of genomic equivalents in the original sample.

As the PCR products from the amplification of single template molecules are substantially homogeneous in sequence, a variety of techniques can be used to characterize the sequence content in each well. Typically, fluorescent probe-based detection methods are particularly useful. For example, to quantify polymorphic regions, a pair of PCR primers and a pair of molecule beacons are designed for each SNP. Typically, molecule beacons are single-stranded oligonucleotides which contain a fluorescent dye and a quencher on their 5' and 3' ends, respectively. Both beacons are identical except for the nucleotide corresponding to the SNP and the fluorescent label (green or red). Typically, molecule beacons include a hairpin structure, which brings the fluorophore closer to the quencher, and do not emit fluorescence when not hybridized to a PCR product. Upon hybridization to their complimentary nucleotide sequences, the quencher is distanced from the fluorophore, resulting in increased fluorescence. Typically, the ratio of fluorescence intensity of two allele-specific beacons with either green or red fluorescence is calculated to determine the allele type in each individual well.

Various digital PCR methods, reagents, and apparatus are known in the art and can be adapted to practice the present invention. See, *e*.*g*., U.S. Pat. Nos. 6,143,496, 6,440,706, 6,753,147, and 7,704,687.

### c. Bridge PCR

In some embodiments, bridge PCR is used to characterize and/or quantify a genomic region. Bridge PCR is also known as solid phase PCR or 2-dimensional PCR. In general, bridge PCR takes place on a solid surface or within a gel, thereby generating a large numbers of "polonies" (polymerase generated colonies) that can be simultaneously sequenced or hybridized with polymorphic probes.

In some embodiments, bridge PCR involves universal amplification reaction, whereby a DNA sample is randomly fragmented, then treated such that the ends of the different fragments all contain the same DNA sequence. For example, DNA fragments can be ligated to universal adapter sequences. Fragments with universal ends can then be amplified in a single reaction with a single pair of amplification primers. Typically, DNA fragments are first individually resolved on a surface, or within a gel, to the single molecule level at each reaction site prior to amplification, which ensures that the amplified molecules form discrete colonies that can then be further analyzed.

In some embodiments, these parallel amplification reactions occur on the surface of a "flow cell" (basically a water-tight microscope slide) which provides a large surface area for many thousands of parallel chemical reactions. The flow cell surface is coated with single stranded oligonucleotides that correspond to the sequences of the adapters ligated during the sample preparation stage. Single-stranded, adapter-ligated fragments are bound to the surface of the flow cell exposed to reagents for polymerase-based extension. Priming occurs as the free/distal end of a ligated fragment "bridges" to a complementary oligo on the surface. Various other solid surface may be used instead of the flow cell surface. For example, solid surface suitable for the invention may include, but are not limited to, latex beads, dextran beads, polystyrene, polypropylene surface, polyacrylamide gel, gold surfaces, glass surfaces and silicon wafers.

Various methods of bridge amplification are well known in the art. See, for example, U.S. Pat. No. 7,115,400, U.S. Publication No. 2009/0226975, and Bing D. H. et al., "Bridge Amplification: A Solid Phase PCR System for the Amplification and Detection of Allelic Differences in Single Copy Genes," Seventh International Symposium on Human Identification (available at the Promega website, promega.com).

Various methods can be used to characterize the sequence content of the amplified nucleic acids generated by bridge PCR. In some embodiments, millions polonies containing amplified nucleic acids may be sequenced by synthesis. For example, Illumina's Solexa Sequencing Technology may be adapted to characterize and quantify a region accordingly to the present invention. For example, a solid surface containing millions of clusters may be subject to sequencing with automated cycles of extension and imaging. The first cycle of sequencing involves first of the incorporation of a single fluorescent nucleotide, followed by high resolution imaging of the entire surface. These images represent the data collected for the first base. Any signal above background identifies the physical location of a cluster (or polony), and the fluorescent emission identifies which of the four bases was incorporated at that position. This cycle is repeated, one base at a time, generating a series of images each representing a single base extension at a specific cluster. Base calls are derived with an algorithm that identifies the emission color over time. Thus, individual sequence read counts attributable to a specific genomic region may be obtained.

In some embodiments, clusters containing amplified nucleic acids may be characterized by hybridization using fluorescent probe. For example, to distinguish and/or quantify polymorphic regions, a pair of molecule beacons can be designed for each SNP. Typically, molecule beacons are single-stranded oligonucleotides which contain a fluorescent dye and a quencher on their 5' and 3' ends, respectively. Both beacons are identical except for the nucleotide corresponding to the SNP and the fluorescent label (green or red). Typically, molecule beacons include a hairpin structure, which brings the fluorophore closer to the quencher, and do not emit fluorescence when not hybridized to a PCR product. Upon hybridization to their complimentary nucleotide sequences, the quencher is distanced from the fluorophore, resulting in increased fluorescence. Typically, the ratio of fluorescence intensity of two allele-specific beacons with either green or red fluorescence is calculated to determine the allele type in each cluster.

### d. Emulsion PCR

In some embodiments, emulsion PCR is used to characterize and/or quantify a genomic region. Typically, emulsion PCR can be used to generate small beads with clonally amplified DNA, i.e., each bead contains one type of amplicon generated from single molecule template by PCR. Exemplary emulsion PCR are described in Dressman et al, Proc. Natl. Acad. Sci. USA., 100, 8817 (Jul. 22, 2003) and Dressman et al. PCT publication WO 2005/010145.

For example, beads coated with capturing oligonucleotides (or colony primers) are mixed with nucleotides with complementary adaptor or tag sequences. An aqueous mix containing all the necessary components for PCR plus primer-bound beads and template DNA are stirred together with an oil/detergent mix to create microemulsions. The aqueous compartments (which may be illustrated as small droplets in an oil layer) contain an average of <1 template molecule and <1 bead. Different templates (*e*.*g*., rare cell population test templates and normal control templates) may be pictured in one or less droplets to represent two template molecules whose sequences differ by one or many nucleotides. The microemulsions are temperature cycled as in a conventional PCR. If a DNA template and a bead are present together in a single aqueous compartment, the bead bound oligonucleotides act as primers for amplification.

Beads made of various materials and in various sizes can be used for the present invention. For example, suitable beads can be magnetic beads, plastic beads, gold particles, cellulose particles, polystyrene particles, to name but a few. Suitable beads can be microparticles in the size range of a few, *e.g.* 1-2, to several hundred, *e.g.* 200-1000 µm diameter. In some embodiments, commercially available controlled-pore glass (CPG) or polystyrene supports are employed as solid phase supports in the invention. Such supports come available with base-labile linkers and initial nucleosides attached, *e*.*g*. Life Technologies (Foster City, Calif.).

In some embodiments, beads containing clonally amplified nucleic acids may be characterized by pyrosequencing *(i.e.,* sequencing by synthesis). For example, beads containing amplified DNA may be subject to a sequencing machine that contains a large number of picoliter-volume wells that are large enough for a single bead, together with enzymes needed for sequencing. In some embodiments, pyrosequencing uses luciferase to generate light as read-out, and the sequencing machine takes a picture of the wells for every added nucleotide and recorded. Sequence read counts attributable to genomic regions may be obtained. Suitable sequencing machines are commercially available, including 454 Life Sciences's Genome Sequencer FLX.

### e. Single Molecule Hybridization With Barcoded Probes

In some embodiments, technology using single molecule hybridization with barcoded probes may be used to characterize and/or quantify a genomic region. In general, such technology uses molecular "barcodes" and single molecule imaging to detect and count specific nucleic acid targets in a single reaction without amplification. Typically, each color-coded barcode is attached to a single target-specific probe corresponding to a genomic region of interest. Mixed together with controls, they form a multiplexed CodeSet. In some embodiments, two probes are used to hybridize each individual target nucleic acid. The Reporter Probe carries the signal; the Capture Probe allows the complex to be immobilized for data collection. After hybridization, the excess probes are removed and the immobilized probe/target complexes may be analyzed by a digital analyzer for data collection. Color codes are counted and tabulated for each target molecule *(e.g.,* a genomic region of interest). Suitable digital analyzers include nCounter^{®}. Analysis System is provided by Nanostring Technologies (on the internet at nanostring.com).

Methods, reagents including molecular "barcodes" an apparatus suitable for nanostring technology are further described in U.S. App. Pub. Nos. 2010/0112710, 2010/0047924, 2010/0015607.

### f. Semiconductor Sequencing

In some embodiments, semiconductor sequencing methods are used to characterize and/or quantify a genomic region. The term "semiconductor sequencing," "semiconductor pH sensitive sequencing," "replication detection sequencing," "direct replication detection sequencing" and "semiconductor replication detection sequencing" as used herein are synonymous and refer generally to the methods of Pourmand and co-workers. See *e.g.,* Pourmand et al., 2006, Proc. Natl. Acad. Sci. USA 103:6466-6470. Exemplary systems for semiconductor sequencing in this context include, *e.g.,* Ion Torrent technology (Life Technologies, Guilford, Conn.). As with other methods of sequencing by synthesis known in the art and described herein, semiconductor sequencing methods are useful to sequence nucleic acid fragments immobilized on a solid support, *i.e.,* a massively parallel array incorporating charge sensors to detect real-time release of proton during DNA replication. Typically, sample DNA is fragmented, *e.g.,* 10-50, 50-150, 50-100, 100-200, 200-400, 400-4000 by sequences, preferably about 100 nucleotides. The sequences are prepared as a library with flanking adapters which are ligated or incorporated by designed PCR primers having the adapter sequences. The library fragments are then clonally amplified using emulsion PCR to form particles coated with template DNA. The particles are deposited on the massively parallel array, which is sequentially contacted with deoxynucleotide triphosphate (dNTP) in the presence of DNA polymerase under conditions suitable for DNA replication. Each incorporation of dNTP into the growing duplex DNA results in the release of a proton, resulting in a change in charge detectable by the charge sensors. Thus, a change in charge (i.e., change in pH) is a specific well of the massively parallel array indicates incorporation of a specific dNTP. No change in charge indicates that the specific dNTP was not incorporated. Multiple proton release (*e.g.,* 2, 3, 4, or more) protons release indicates that a corresponding sequence of a specific dNTP was incorporated. Correlation of the change in charge of each well in the massively parallel array with the presence of a specific dNTP thus provides the sequence of the DNA sample.

Unidirectional sequencing requires only one fusion primer pair and will produce reads from only one end of the amplicon. Bidirectional sequencing can be conducted for optimal results, producing high quality reads from both ends and across the full length of the amplicons.

The length of the target regions can be optimized. For example, with a typical read length of 100 nucleotides, the first 20-25 nucleotides of sequence correspond to the target specific sequence of the PCR primers and will not produce informative data. Accordingly, in some cases, a target region of about 75 bp is employed.

Depth of coverage requirements depend on the expected frequency of mutation with a sample and dictate the number of amplicons that are included given a fixed amount of sequence throughput per massively parallel array. For example, for germ-line mutations that follow standard Mendelian inheritance patterns, either 100% or 50% of the reads are expected to contain a given sequence variant. It is believed that in these cases an average depth of coverage of 100-200x provides a sufficient number of reads to detect variants with statistical confidence. For high confidence detection of somatic mutations present at variable and typically low frequencies in heterogeneous samples, *e*.*g*., heterogeneous cancer samples, deeper coverage of up to 1000-2000x is thought to be required.

Methods, reagents and apparatus are further described in the seminal work of Pourmand and co-workers, *e*.*g*., U.S. Pat. No. 7,785,785.

### g. Detectable Entities

Any of a wide variety of detectable agents can be used in the practice of the present invention. Suitable detectable agents include, but are not limited to: various ligands, radionuclides; fluorescent dyes; chemiluminescent agents (such as, for example, acridinum esters, stabilized dioxetanes, and the like); bioluminescent agents; spectrally resolvable inorganic fluorescent semiconductors nanocrystals (*i.e.,* quantum dots); microparticles; metal nanoparticles (*e*.*g*., gold, silver, copper, platinum, etc.); nanoclusters; paramagnetic metal ions; enzymes; colorimetric labels (such as, for example, dyes, colloidal gold, and the like); biotin; dioxigenin; haptens; and proteins for which antisera or monoclonal antibodies are available.

In some embodiments, the detectable moiety is biotin. Biotin can be bound to avidins (such as streptavidin), which are typically conjugated (directly or indirectly) to other moieties (*e*.*g*., fluorescent moieties) that are detectable themselves.

In addition to exemplary detectable entities described in connection with various methods described herein, below are described some non-limiting examples of other detectable moieties.

### i. Fluorescent Dyes

In certain embodiments, a detectable moiety is a fluorescent dye. Numerous known fluorescent dyes of a wide variety of chemical structures and physical characteristics are suitable for use in the practice of the present invention. A fluorescent detectable moiety can be stimulated by a laser with the emitted light captured by a detector. The detector can be a charge-coupled device (CCD) or a confocal microscope, which records its intensity.

Suitable fluorescent dyes include, but are not limited to, fluorescein and fluorescein dyes (*e*.*g*., fluorescein isothiocyanine or FITC, naphthofluorescein, 4',5'-dichloro-2',7'-dimethoxyfluorescein, 6-carboxyfluorescein or FAM, *etc*.), carbocyanine, merocyanine, styryl dyes, oxonol dyes, phycoerythrin, erythrosin, eosin, rhodamine dyes (*e*.*g*., carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine (TMR), etc.), coumarin and coumarin dyes (*e.g.,* methoxycoumarin, dialkylaminocoumarin, hydroxycoumarin, aminomethylcoumarin (AMCA), etc.), Oregon Green Dyes (*e.g.,* Oregon Green 488, Oregon Green 500, Oregon Green 514, etc.), Texas Red, Texas Red-X, SPECTRUM RED™, SPECTRUM GREEN™, cyanine dyes (*e*.*g*., CY-3™, CY-5™, CY-3.5™, CY-5.5™, etc.), ALEXA FLUOR™ dyes (*e*.*g*., ALEXA FLUOR™ 350, ALEXA FLUOR™ 488, ALEXA FLUOR™ 532, ALEXA FLUOR™ 546, ALEXA FLUOR™ 568, ALEXA FLUOR™ 594, ALEXA FLUOR™ 633, ALEXA FLUOR™ 660, ALEXA FLUOR™ 680, etc.), BODIPY™ dyes (*e*.*g*., BODIPY™ FL, BODIPY™ R6G, BODIPY™ TMR, BODIPY™ TR, BODIPY™ 530/550, BODIPY™ 558/568, BODIPY™ 564/570, BODIPY™ 576/589, BODIPY™ 581/591, BODIPY™ 630/650, BODIPY™ 650/665, etc.), IRDyes (*e.g.,* IRD40, IRD 700, IRD 800, etc.), and the like. For more examples of suitable fluorescent dyes and methods for coupling fluorescent dyes to other chemical entities such as proteins and peptides, see, for example, "The Handbook of Fluorescent Probes and Research Products", 9th Ed., Molecular Probes, Inc., Eugene, Oreg. Favorable properties of fluorescent labeling agents include high molar absorption coefficient, high fluorescence quantum yield, and photostability. In some embodiments, labeling fluorophores exhibit absorption and emission wavelengths in the visible (i.e., between 400 and 750 nm) rather than in the ultraviolet range of the spectrum (i.e., lower than 400 nm).

A detectable moiety may include more than one chemical entity such as in fluorescent resonance energy transfer (FRET). Resonance transfer results an overall enhancement of the emission intensity. For instance, see Ju et. al., (1995), Proc. Nat'l Acad. Sci. (USA), 92:4347. To achieve resonance energy transfer, the first fluorescent molecule (the "donor" fluor) absorbs light and transfers it through the resonance of excited electrons to the second fluorescent molecule (the "acceptor" fluor). In one approach, both the donor and acceptor dyes can be linked together and attached to the oligo primer. Methods to link donor and acceptor dyes to a nucleic acid have been described previously, for example, in U.S. Pat. No. 5,945,526 to Lee et al.*.* Donor/acceptor pairs of dyes that can be used include, for example, fluorescein/tetramethylrohdamine, IAEDANS/fluroescein, EDANS/DABCYL, fluorescein/fluorescein, BODIPY FL/BODIPY FL, and Fluorescein/QSY 7 dye. See, *e.g.,* U.S. Pat. No. 5,945,526 to Lee et al. Many of these dyes also are commercially available, for instance, from Molecular Probes Inc. (Eugene, Oreg.). Suitable donor fluorophores include 6-carboxyfluorescein (FAM), tetrachloro-6-carboxyfluorescein (TET), 2'-chloro-7'-phenyl-1,4-dichloro-6-carboxyfluorescein (VIC), and the like.

### ii. Enzymes

In certain embodiments, a detectable moiety is an enzyme. Examples of suitable enzymes include, but are not limited to, those used in an ELISA, e.g., horseradish peroxidase, beta-galactosidase, luciferase, alkaline phosphatase, etc. Other examples include betaglucuronidase, beta-D-glucosidase, urease, glucose oxidase, etc. An enzyme may be conjugated to a molecule using a linker group such as a carbodiimide, a diisocyanate, a glutaraldehyde, and the like.

### iii. Radioactive Isotopes

In certain embodiments, a detectable moiety is a radioactive isotope. For example, a molecule may be isotopically-labeled (i.e., may contain one or more atoms that have been replaced by an atom having an atomic mass or mass number different from the atomic mass or mass number usually found in nature) or an isotope may be attached to the molecule. Non-limiting examples of isotopes that can be incorporated into molecules include isotopes of hydrogen, carbon, fluorine, phosphorous, copper, gallium, yttrium, technetium, indium, iodine, rhenium, thallium, bismuth, astatine, samarium, and lutetium (*e*.*g*., ³H, ¹³C, ¹⁴C, ¹⁸F, ¹⁹F, ³²P, ³⁵S, ⁶⁴Cu, ⁶⁷Cu, ⁶⁷Ga, ⁹⁰Y, ⁹⁹mTc, ¹¹¹¹In, ¹²⁵I, ¹²³I, ¹²⁹I, ¹³¹I, ¹³⁵I, ¹⁸⁶Re, ¹⁸⁷Re, ²⁰¹T1, ²¹²Bi, ²¹³Bi, ²¹¹At, ¹⁵³Sm, ¹⁷⁷Lu).

In some embodiments, signal amplification is achieved using labeled dendrimers as the detectable moiety (see, *e.g.,* Physiol Genomics, 3:93-99, 2000). Fluorescently labeled dendrimers are available from Genisphere (Montvale, N.J.). These may be chemically conjugated to the oligonucleotide primers by methods known in the art.

### 7. Comparing Amplified and Unamplified Nucleic Acid Sequences

The detected presence or absence of genomic mutations (e.g., SNPs) at the same locations in the genomic DNA are compared between the amplified test genomic DNA from the rare cell population (e.g., CTC) and unamplified control genomic DNA comprising normal somatic genomic DNA. This step comprises comparing the amplified genomic sequences obtained by amplifying two or more portions of genomic DNA from rare cell population with one DNA polymerase multiple times or with two or more different DNA polymerases with unamplified normal somatic control genomic sequences at the same nucleotide positions in the genome. Identification of a nucleotide polymorphism, e.g., single polynucleotide polymorphism (SNP), that is identical in the amplified genomic sequences (either multiple times by the same DNA polymerase or by the two or more different DNA polymerases), but different from a nucleotide polymorphism at the same nucleotide position in the unamplified control genomic DNA from the control cells verify the presence of a genomic mutation (e.g., SNP) in cells of the rare cell population.

### EXAMPLES

The following examples are offered to illustrate, but not to limit the claimed invention.

### Example 1

### Rare Cell Analysis Without Whole Genome Amplification By Massively Parallel Sequencing.

### MATERIALS AND METHODS

*DNA*/*Cell Template Construction*. For amplified genome experiments, purified genomic DNA was combined prior to amplification reactions. For each WGA reaction 2.4 ng of genomic DNA (∼400 cell equivalents based upon about 6 pg/cell) [5] was used.

For direct sequencing libraries (DSL), cell pellets were processed to liberate DNA and then directly used in the library construction process without further purification. For DSL experiments, all reactions utilized about 400 cells. This number is not arbitrary but is based upon the average performance of the Cynvenio Biosystems CTC isolation platform (*See, e.g.,* U.S. Patent Publication Nos. 2011/0137018; 2011/0127222; 2011/0003303; 2010/0317093; and 2009/0053799). The purity of CTCs recovered depends, in part, upon the patient blood sample and their circulating tumor load. When there are many CTCs in a sample, purity can be greater than 60%; frequently there are only a few CTCs per ml blood and the purity of the recovered CTC pellet is about 1 - 2%.

Whole Blood was treated with Versalyse (Beckman Coulter) to produce White Blood Cells (WBC), they were subsequently counted with a hemacytometer. Tumor cell lines A549 and MCF7 were obtained from ATCC and maintained for a maximum of 10 generations.

### Spike-in construction-cell dilutions.

White Blood Cells were diluted to 2 cells per 1 µL in a total of 20 mL of elution buffer. A549 cells were diluted to 2 cells per 1 µL in 5 mL of elution buffer. MCF-7 cells were diluted to 2 cells per 1 µL in 5 mL of elution buffer.

For mixtures of tumor cells, the cells were co-diluted to 1 cell per 5 µL (MCF-7) and 1 cell per 10 µL (A549) respectively in 5 mL elution buffer = (TumorDilution-A: TD-A). A portion of this mixture was then serial diluted 1:2 times giving mixtures of 1 MCF-7 per 10 and 1 A549 per 20 µL = (TumorDilution-B: TD-B), and 1:2 times yielding 1 MCF-7 per 20 and 1 A549 per 40 µL = (TumorDilution-C: TD-C).

### Cell Pellet construction.

Cell concentrations: White Blood Cells alone: 100 µL (200 WBC).
A549 cells alone: 100 µL (200 A549).
MCF-7 cells alone: 100 µL (200 MCF-7).

Cell Pellet mixtures consisted of:
20/40 WBC+T: 200 µL WBC + 200 µL TD-A (400 WBC + 40 MCF-7 + 20 A549).
10/20 WBC+T: 200 µL WBC + 200 µL TD-B (400 WBC + 20 MCF-7 + 10 A549).
5/10 WBC+T: 200 µL WBC + 200 µL TD-C (400 WBC + 10 MCF-7 + 5 A549).

These cell mixtures were then pelleted in Thermo Scientific centrifuge and spun at 21,000 x G for 5 minutes. Residual supernatant was removed with a Drummond capillary pipette. 6.0 µL of digestion buffer was added to the bottom of the tube. Cell Pellets/tubes were sonicated in sonicator for 10 seconds, briefly spun to collect the contents in the bottom of the tube and placed into thermal cycler (MJ Research) equipped with 0.5 mL tube block for 3 hours at 55°C, followed by 1 hour at 70°C to heat inactivate the enzyme, using the heated lid option on the thermal cycler's bonnet.

*Whole Genomic Amplification.* All whole genome amplifications were carried out following manufactures instructions supplied with the commercially available kits. 2.4 ng of genomic DNA (about 400 cell equivalents) was used for each WGA reaction according to manufactures recommendation.

*WGA reactions*. Amplifications were carried out independently using one of the following commercially available WGA kits, either:
Phi29/GenomiPhi DNA Amplification Kit (GE Healthcare)
Rubicon PicoPlex NGS Kit (Rubicon Genomics).

*WGA Sequencing Library construction*. For WGA libraries, 41.3 ng of WGA DNA and 41.3 gDNA for unamplified genomic controls (about 6800 cell equivalents [5]) was utilized for each sequencing library. Thus the gWBC and gWBC+T controls genomic libraries used identical amounts of genomic DNA for control library construction.

Using Life Technologies AmpliSeq 2.0 kit and AmpliSeq Cancer Hot Spot Panel kits, PCR master mix was generated according to manufacturer specifications. 14 µL of the master mix was added to the final digested product, vortexed gently to mix, and briefly spun down to collect the PCR reaction mix. This was then transferred to a new Axigen low-bind 200 µL PCR tube, and thermal cycled according to manufacturer specifications in ABI thermal cycler 2200.

All other procedures of library creation were carried out according to manufacturer specifications including the post-amplification and clean-up for analysis on an Agilent BioAnalyzer.

*Primary Ampliseq library qualification.* BioAnalyzer smear analysis was done to determine the concentration of the AmpliSeq specific products between 125 and 300 bp. This concentration was used to generate a library dilution factor specific to each of the 15 libraries. Library dilutions were generated immediately prior to their use by pipetting 4 µL of library into the appropriate dilution of Nuclease-Free water (provided in the kit) in an Eppendorf Low-Bind 1.5 mL snap-cap tube. Diluted Library was vortexed to mix and briefly spun down to collect the sample.

*Ion Sphere Particle (ISP) construction*. Diluted Library was used to generate emulsion PCR on the ISP particles in the OneTouch200 V2 kit according to the manufacturer's instructions. Post-emulsion PCR clean-up and enrichment was done on the OneTouchES machine following manufacturer's instructions with freshly prepared MyOne beads and Melt-Off solutions.

ISPs were collected, primer annealed, polymerase bound, and loaded onto a fresh 316 chip according to manufacturer's instructions. PGM sequencing was then performed and data exported and analyzed with the NGEN and Seqman Pro software from DNAStar.

### RESULTS

The first experiment, used limiting amounts of A549 DNA mixed with genomic DNA from a healthy donor (isolated from their white blood cells) as a basic template to test the effect of WGA. In all cases, A549 DNA was spiked into the WBC DNA at a ratio of about 20 genomes A459: about 400 WBC genomes. 2.4 ng is equal to about 400 cell equivalents of DNA (based upon the published 6 pg DNA/diploid human cell [5]). This 2.4 ng was then amplified either using Phi29 (also called Φ29) (GE Healthcare) or Rubicon PicoPlex NGS (Rubicon Genomics). 41.3 ng of amplified template was used to construct each Ion Torrent Cancer Ampliseq library which were then subjected to sequencing on a Ion Torrent PGM.

All reads were first aligned top down to Human GRCh37_p2 from NCBI, using the NGEN assembler from DNAStar. After alignment, Chromosomes 3, 12 and 19 were analyzed for informative reads from PIK3CA, KRAS, and STK11. These genes were secondarily analyzed using SNP variant caller included in the Seqman Pro application from DNAStar. Table 1 shows a compilation of these reads.

**TABLE 1**

| **MID** | **Contiq ID** | **Ref Pos** | **Impact** | **SNP %** | **dbSNP ID** | **Feature Name** | **DNA Change** | **Protein Change** | **Depth** | |
|---|---|---|---|---|---|---|---|---|---|---|
| gWBC | NC_000003 | 178938877 | Non Synon | 16.90% | 3729687 | PIK3CA | c.2119G>A | E707K | 16839 | |
| gWBC+T | NC_000003 | 178938877 | Non Synon | 17.30% | 3729687 | PIK3CA | c.2119G>A | E707K | 23534 | |
| Phi_a | NC_000003 | 178938877 | Non Synon | 31.90% | 3729687 | PIK3CA | c.2119G>A | E707K | 18106 | |
| Phi_b | NC_000003 | 178938877 | Non Synon | 22.90% | 3729687 | PIK3CA | c.2119G>A | E707K | 15983 | |
| Phi_c | NC_000003 | 178938877 | Non Synon | 24.70% | 3729687 | PIK3CA | c.2119G>A | E707K | 15575 | |
| Phi_d | NC_000003 | 178938877 | Non Synon | 24.20% | 3729687 | PIK3CA | c.2119G>A | E707K | 13253 | |
| Rub_a | NC_000003 | 178938877 | Non Synon | 0.00% | 3729687 | PI K3CA | c.2119G>A | E707K | 0 | * |
| Rub_b | NC_000003 | 178938877 | Non Synon | 0.00% | 3729687 | PI K3CA | c.2119G>A | E707K | 0 | * |
| Rub_c | NC_000003 | 178938877 | Non Synon | 0.00% | 3729687 | PI K3CA | c.2119G>A | E707K | 0 | * |
| | | | | | | | | | | |
| gWBC+T | NC_000012 | 25398285 | Non Synon | 6.00% | | KRAS [1] | c.34C>T | G12S | 7958 | |
| Phi_a | NC_000012 | 25398285 | Non Synon | 1.20% | | KRAS [1] | c.34C>T | G12S | 1298 | |
| Phi_c | NC_000012 | 25398285 | Non Synon | 1.50% | | KRAS [1] | c.34C>T | G12S | 2474 | |
| Phi_d | NC_000012 | 25398285 | Non Synon | 2.00% | | KRAS [1] | c.34C>T | G12S | 1799 | |
| Rub_a | NC_000012 | 25398285 | Non Synon | 0.00% | | KRAS [1] | c.34C>T | G12S | 0 | * |
| Rub_b | NC_000012 | 25398285 | Non Synon | 0.00% | | KRAS [1] | c.34C>T | G12S | 0 | * |
| Rub_c | NC_000012 | 25398285 | Non Synon | 0.00% | | KRAS [1] | c.34C>T | G12S | 0 | * |
| | | | | | | | | | | |
| gWBC+T | NC_000019 | 1207021 | Nonsense | 5.50% | | STK11 | c.109C>T | Q37. | 8751 | |
| Rub_a | NC_000019 | 1207021 | Nonsense | 1.40% | | STK11 | c.109C>T | Q37. | 22063 | |
| Rub_b | NC_000019 | 1207021 | Nonsense | 5.20% | | STK11 | c.109C>T | Q37. | 17153 | |
| Rub_c | NC_000019 | 1207021 | Nonsense | 3.70% | | STK11 | c.109C>T | Q37. | 17796 | |
| Phi_a | NC_000019 | 1207021 | Nonsense | 0.00% | | STK11 | c.109C>T | Q37. | 6503 | # |
| Phi_b | NC_000019 | 1207021 | Nonsense | 0.00% | | STK11 | c.109C>T | Q37. | 8213 | # |
| Phi_c | NC_000019 | 1207021 | Nonsense | 0.00% | | STK11 | c.109C>T | Q37. | 5231 | # |
| Phi_d | NC_000019 | 1207021 | Nonsense | 0.00% | | STK11 | c.109C>T | Q37. | 3124 | # |

| | | | | | | | | | | |
|---|---|---|---|---|---|---|---|---|---|---|
| *NO Rubicon reads at all; locus specific bias #YES STK11 WGA reads. But No STK11 Q37* reads; allelic biasing | | | | | | | | | | |

The Chromosome 3 SNP, PIK3CA, E707K (dbSNP ID #3729687) was present in all unamplified genomic WBC samples. Both gWBC and gWBC+T showed similar representation of allele frequencies (16.9% vs 17.3%). Using the Phi29 amplification protocol this SNP was detected at higher frequencies (24.2% - 31.9%). However, using the Rubicon protocol the allele frequencies were 0% as there were no reads from this locus.

For Chromosome 12, KRAS G12S mutation was successfully detected in both unamplified genomic DNA and Phi29 amplified DNA, but not in Rubicon amplified DNA. Once again there were NO KRAS reads at all in Rubicon amplified libraries.

Chromsome 19 showed a distinctly different pattern as the STK11 Q37* mutation was easily detectable in Rubicon amplified DNA but not in Phi29 amplified DNA. Remarkably for both Rubicon amplified and Phi29 amplified DNA, reads from the STK11 locus were plentiful, but there were no informative reads for the mutated Q37* allele in the Phi29 libraries. The results are shown in Figures 1A-D and in Figures 2a-d.

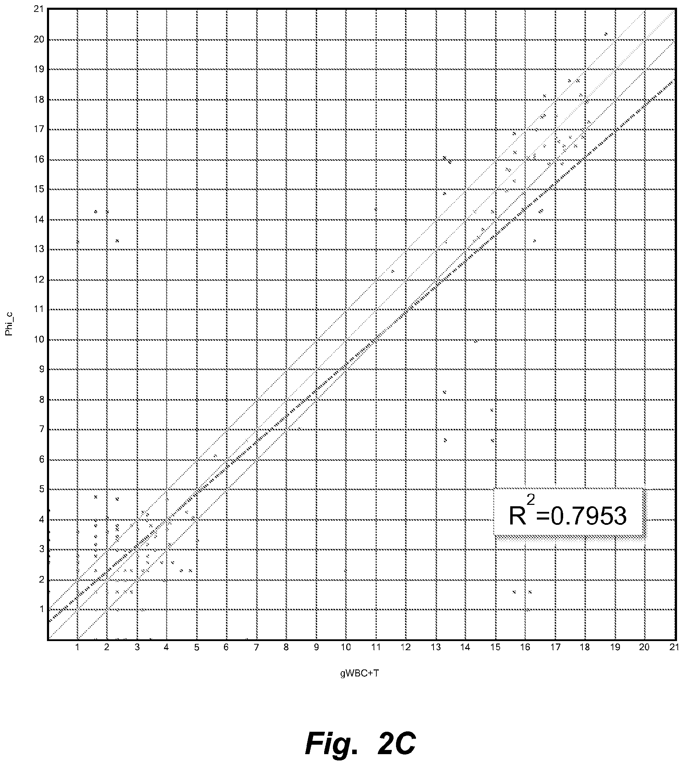

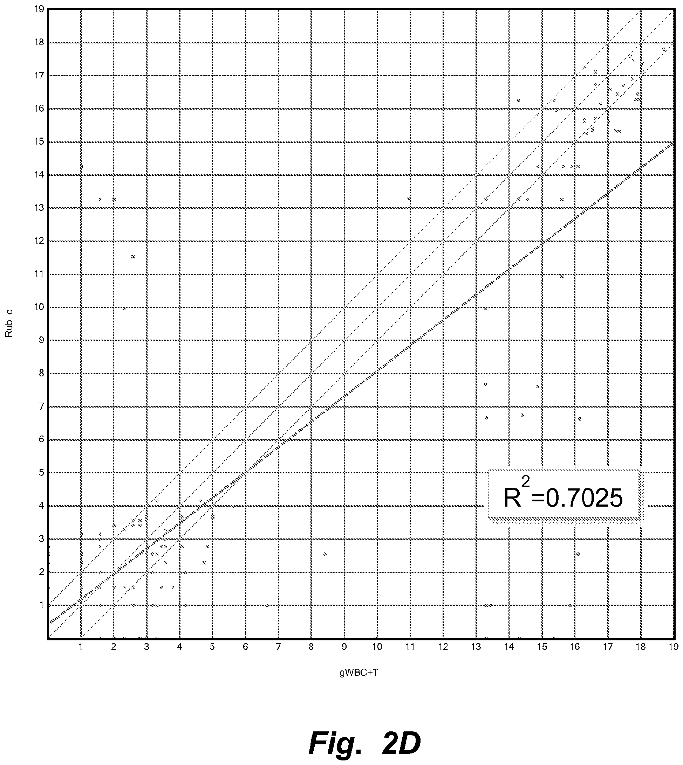

The second set of experiments were designed to test the utility of Direct Sequencing Libraries (DSL) directly derived from cell pellets. Analysis using cell mixtures and unamplified templates is presented in Table 2 and Figure 2c & 2d. Using our proprietary method for isolating genomic DNA from small numbers of cells, we prepared Ion Torrent Ampliseq libraries from mixtures of A549 cells:MCF7 cells: WBC. The libraries were constructed either from (a) 5 A549 cells: 10 MCF7 cells:400 WBC; (b) 10 A549 cells:20 MCF7 cells:400 WBC; or (c) from 20 A549 cells:40 MCF7 cells:400 WBC. Spike in libraries were prepared in triplicate for both 5/10/400 cells or 20/40/400 cell inputs, only one 10/20/400 cell library was evaluated. Either pure A549 or MCF7 cells were used to prepare two control libraries each. Single donor WBC libraries were prepared in duplicate.

**TABLE 2**

| **MID** | **Contiq ID** | **Ref Pos** | **Impact** | **SNP %** | **dbSNP ID** | **Feature Name** | **DNA Change** | **Protein Change** | **Depth** |
|---|---|---|---|---|---|---|---|---|---|
| MCF7a | NC_000003 | 178936091 | Non Synonymous | 45.20% | 104886003 | PIK3CA | c.1633G>A | E545K | 10468 |
| MCF7b | NC_000003 | 178936091 | Non Synonymous | 40.50% | 104886003 | PIK3CA | c.1633G>A | E545K | 11634 |
| 5a | NC_000003 | 178936091 | Non Synonymous | 3.00% | 104886003 | PIK3CA | c.1633G>A | E545K | 18534 |
| 5b | NC_000003 | 178936091 | Non Synonymous | 3.90% | 104886003 | PIK3CA | c.1633G>A | E545K | 22598 |
| 5c | NC_000003 | 178936091 | Non Synonymous | 1.20% | 104886003 | PIK3CA | c.1633G>A | E545K | 19165 |
| 10 | NC_000003 | 178936091 | Non Synonymous | 11.30% | 104886003 | PIK3CA | c.1633G>A | E545K | 18158 |
| 20a | NC_000003 | 178936091 | Non Synonymous | 9.20% | 104886003 | PIK3CA | c.1633G>A | E545K | 18932 |
| 20b | NC_000003 | 178936091 | Non Synonymous | 10.90% | 104886003 | PIK3CA | c.1633G>A | E545K | 21608 |
| 20c | NC_000003 | 178936091 | Non Synonymous | 4.80% | 104886003 | PIK3CA | c.1633G>A | E545K | 9211 |
| | | | | | | | | | |
| MCF7a | NC_000003 | 178938877 | Non Synonymous | 27.70% | 3729687 | PIK3CA | c.2119G>A | E707K | 19985 |
| MCF7b | NC_000003 | 178938877 | Non Synonymous | 24.60% | 3729687 | PIK3CA | c.2119G>A | E707K | 22519 |
| 5a | NC_000003 | 178938877 | Non Synonymous | 1.20% | 3729687 | PIK3CA | c.2119G>A | E707K | 20364 |
| 5b | NC_000003 | 178938877 | Non Synonymous | 1.30% | 3729687 | PIK3CA | c.2119G>A | E707K | 19223 |
| 5c | NC_000003 | 178938877 | Non Synonymous | 1.80% | 3729687 | PIK3CA | c.2119G>A | E707K | 20024 |
| 10 | NC_000003 | 178938877 | Non Synonymous | 7.40% | 3729687 | PIK3CA | c.2119G>A | E707K | 18035 |
| 20a | NC_000003 | 178938877 | Non Synonymous | 4.70% | 3729687 | PI K3CA | c.2119G>A | E707K | 18511 |
| 20b | NC_000003 | 178938877 | Non Synonymous | 6.80% | 3729687 | PI K3CA | c.2119G>A | E707K | 21302 |
| 20c | NC_000003 | 178938877 | Non Synonymous | 2.90% | 3729687 | PIK3CA | c.2119G>A | E707K | 10895 |
| | | | | | | | | | |
| A549a | NC_000012 | 25398285 | Non Synonymous | 99.70% | | KRAS [1] | c.34C>T | G12S | 9437 |
| A549b | NC_000012 | 25398285 | Non Synonymous | 99.70% | | KRAS [1] | c.34C>T | G12S | 10062 |
| 5a | NC_000012 | 25398285 | Non Synonymous | 2.80% | | KRAS [1] | c.34C>T | G12S | 10105 |
| 5b | NC_000012 | 25398285 | Non Synonymous | 3.80% | | KRAS [1] | c.34C>T | G12S | 12884 |
| 5c | NC_000012 | 25398285 | Non Synonymous | 3.70% | | KRAS [1] | c.34C>T | G12S | 9911 |
| 10 | NC_000012 | 25398285 | Non Synonymous | 6.20% | | KRAS [1] | c.34C>T | G12S | 9128 |
| 20a | NC_000012 | 25398285 | Non Synonymous | 14.50% | | KRAS [1] | c.34C>T | G12S | 10546 |
| 20b | NC_000012 | 25398285 | Non Synonymous | 8.40% | | KRAS [1] | c.34C>T | G12S | 10410 |
| 20c | NC_000012 | 25398285 | Non Synonymous | 9.10% | | KRAS [1] | c.34C>T | G12S | 5306 |
| | | | | | | | | | |
| A549a | NC_000019 | 1207021 | Nonsense | 99.00% | | STK11 | c.109C>T | Q37. | 10024 |
| A549b | NC_000019 | 1207021 | Nonsense | 99.80% | | STK11 | c.109C>T | Q37. | 12150 |
| 5a | NC_000019 | 1207021 | Nonsense | 1.60% | | STK11 | c.109C>T | Q37. | 14072 |
| 5b | NC_000019 | 1207021 | Nonsense | 1.70% | | STK11 | c.109C>T | Q37. | 19062 |
| 5c | NC_000019 | 1207021 | Nonsense | 2.00% | | STK11 | c.109C>T | Q37. | 13046 |
| 10 | NC_000019 | 1207021 | Nonsense | 5.50% | | STK11 | c.109C>T | Q37. | 13539 |
| 20a | NC_000019 | 1207021 | Nonsense | 10.80% | | STK11 | c.109C>T | Q37. | 11682 |
| 20b | NC_000019 | 1207021 | Nonsense | 9.80% | | STK11 | c.109C>T | Q37. | 11483 |
| 20c | NC_000019 | 1207021 | Nonsense | 7.40% | | STK11 | c.109C>T | Q37. | 9414 |

For chromosome 3, The E707K (dbSNP ID#3729687) was not detected in WBC or A549 (different WBC donor), but samples containing MCF7 cells did contain the E707K SNP. Similarly, when as few as 10 MCF7 cells were present in a cell pellet, the heterozygous E545K (dbSNP 104886003) allele could be detected.

For chromosome 12, the homozygous KRAS G12S alleles were detected in all samples containing A549 cells (Table 2). Libraries from 20 A549 as well as libraries from 5 A549 cells all showed G12S reads.

Similarly, for chromosome 19 the homozygous mutations for STK11 Q37* were detectable (Table 2). Libraries from 20 A549 as well as libraries from 5 A549 cells all showed Q37* reads.

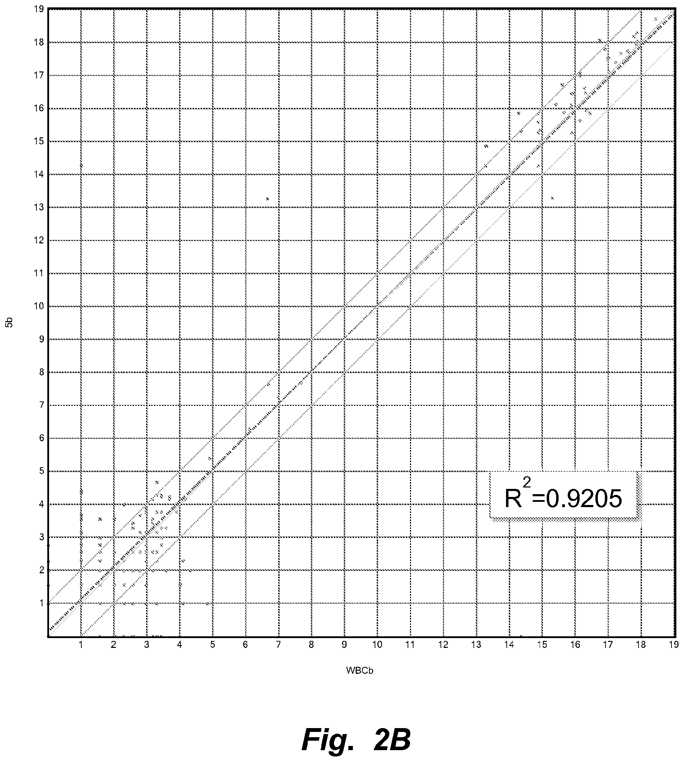

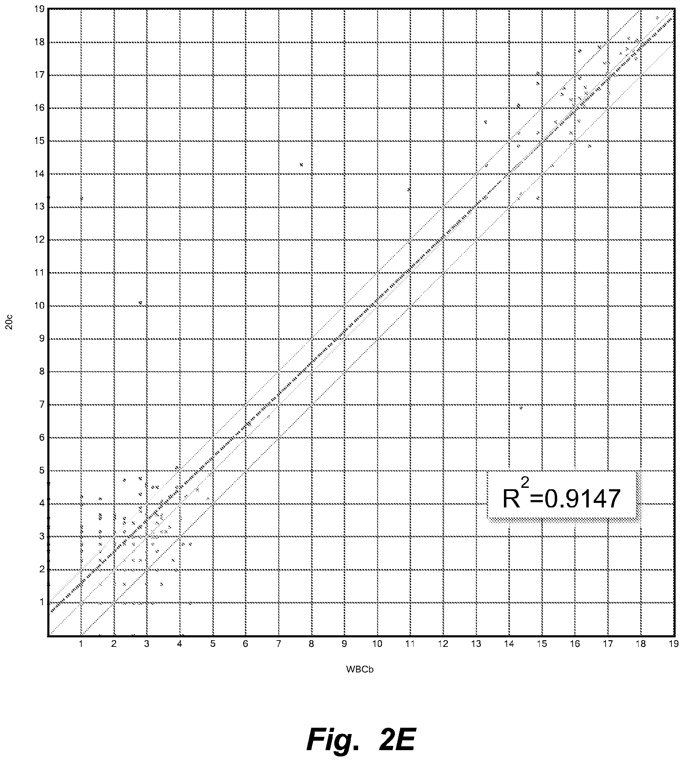

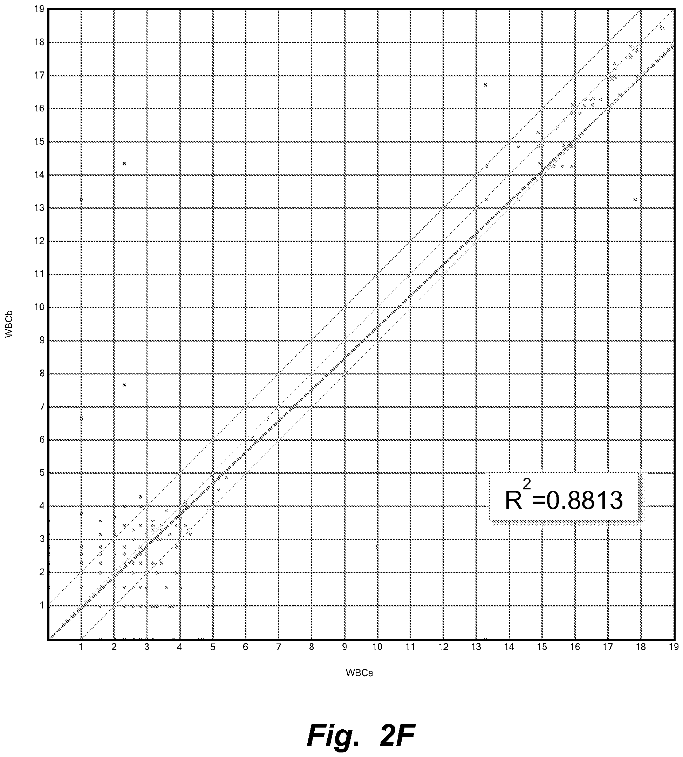

Figure 2, panels 2c, 2d, Panel 2c, scatter plot comparison between two independent WBC libraries, yield a R2 = 0.8813. Panel 2d, scatter analysis of WBC vs 20/40 cell spike genomic libraries yields R2 = 0.9147. In all four cases the number of neutral synonymous substitutions are similar (lower left hand quadrant), for the two amplified libraries the number of major alterations is increased (upper right hand quadrant).

### DISCUSSION

The focus of our development plan has been to devise a stable, robust platform for isolation of CTCs (rare cells) with sufficient purity for molecular analysis, specifically Next Generation Sequencing [6]. The Cynvenio Biosystems CTC purification platform typically produces about 400 cells per run, and depending on the patient tumor load with a purity ranging from about 1% to greater than 60%. The purity of the samples, however, is only one consideration for successful molecular analysis. Further considerations include the amount of available template and the sequencing strategy. Whole genome and most exomic approaches are rather demanding with respect to template quantity [7, 8, 9].

In clinical samples, the number of CTCs can vary quite dramatically depending upon the type and stage of cancer. Any robust sequencing library method must be engineered to handle samples where the number of CTCs is less than 10 / ml. For early stage cancers, frequently the CTC concentration is 2 -3 cells per ml [10, 11, 12, 13, 14].

Our device technology can purify such samples. However, in light of the extreme limit of CTC number it was important to develop strategies which could support meaningful molecular analysis of CTC (rare cell) samples. Thus one of our first molecular questions has been to determine which SNP sequencing strategy is compatible with the constraints of CTC biology.

Given the attractive potential of WGA in extending the availability of limiting amounts of template, we decided to examine the library representation in libraries constructed from WGA amplified samples.

In all WGA libraries we observed significant library bias.

In Table 1, where unamplified genomic DNA is compared to WGA amplified genomic DNA, two different types of bias were observed. On chromosome 3, the E707K PIK3CA mutation was not detectable after Rubicon WGA. Similarly, no G12S KRAS mutation was observed after Rubicon amplification, in both of these cases, no reads were detected on Chromosome 3 or 12 at the PIK3CA or KRAS loci after Rubicon WGA library construction. Based on both of these examples, we define this observed type of WGA bias to be called: "Locus-Specific Loss." This bias is typified by total lack of representation or "holes" in the amplified genome.

A different class of bias was observed on Chromosome 19 for the STK11 mutation Q37* where significant amplification and corresponding sequencing reads of this region was observed for both WGA methods but using the Phi29 WGA protocol no Q37* allele was observed. The Q37* allele was detected in unamplified samples and after Rubicon amplification, but genomic samples amplified using the Phi29 WGA system showed no Q37* allele. We call this type of bias: "Allele-Specific Loss." This bias is typified by lack of representation for one allele or another and is not detectable by a "hole" in the amplified genome.

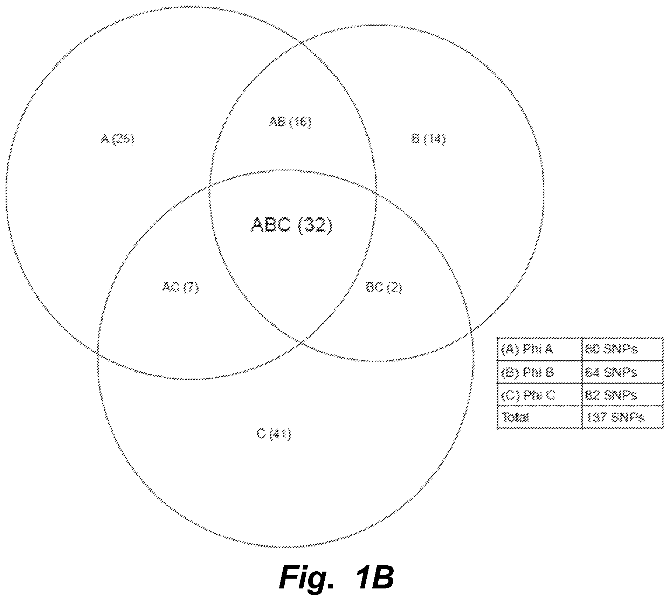

In addition to our concerns regarding biasing after WGA, we were also concerned about artifactual mutations introduced by WGA. To measure the artifactual mutational spectrum, Boolean analysis was undertaken by VENN diagramming of unamplified and amplified samples. In Figure 1a, VENN diagram of unamplified WBC and WBC+Tumor cell spikes are compared showing that for these samples the unamplified samples are largely concordant with 59% overlap. In Figure 1b, we compare two amplified libraries to one unamplified library. The number of SNPs in common is quite different as the three libraries only shared 3% of their SNPs. Even more striking where unamplified libraries showed only 52 SNPs, the Phi29 library showed 161 SNPs and the Rubicon library showed 707 SNPs. As all the libraries shared the same starting material the non-overlapping SNPs must have been the result of the amplification process.

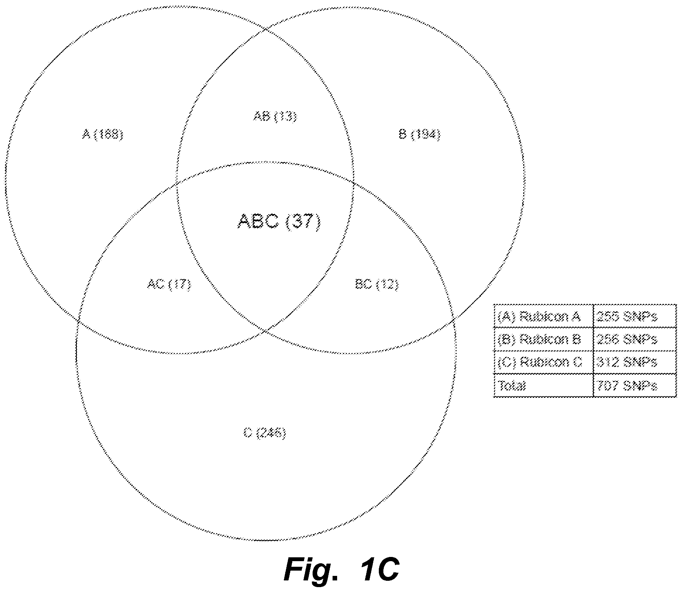

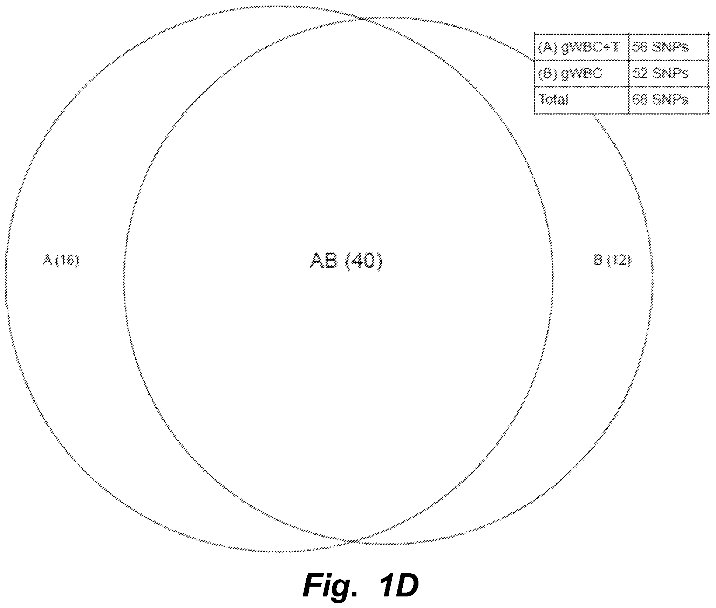

Precision experiments measuring the reproducibility of SNP content in replicate WGA reaction libraries showed largely discordant results. In Figure 1c & 1d three Phi29 libraries showed only 23% concordance for their SNP content. Even more troublesome, three Rubicon libraries showed only 5% SNP concordance. Thus the amplification process, as measured in these experiments, shows significant loss of representation and significant SNP artifacts.

Surveying all SNP variation was further accomplished by scatter plot analysis. In a comparison between a genomic WBC library to a Phi29 WGA library the linear coefficient of variation (R2) shows R2 = ~0.8 concordance (Figure 2a). When comparing a WBC library to a Rubicon amplified library the concordance was R2 = ~0.7 (Figure 2b). This showed once again that the method of amplification has an impact on SNP content. This is not desired or expected as the minor contribution of the spiked cells is no more than 1%. Skewed SNP content is not due to the spike contribution, rather the method of amplification.

In view of the concerns of library bias, we wished to pursue different library construction practices to enable reliable SNP analysis from CTC (rare cell) samples. Given the good concordance of SNP content between control genomic cell samples (Figure 1a) we decided to investigate direct amplicon sequencing of rare cell isolates. We prepared cell pellet samples consisting of tumor cell spikes at numbers consistent with real world patient samples. Libraries were constructed from 5 A549 cells/10 MCF7 cells, 10 A549 cells/20 MCF7 cells, and 20 A549 cells/40 MCF7 cells in a background of 400 WBC.

Table 2 shows the results of these experiments for Chromosome 3, 12, 19 and for genes PIK3CA E545K, E707K, KRAS G12S, and STK11 Q37*.

At the outset we determined there was no blatant biasing of the direct genomic amplicon libraries at chromosomes 3, 12 or 19 as seen for WGA libraries. Every mutant allele at Chromosome 3, 12 or 19 was present in the low and high spike number libraries at frequencies that were consistent with the cell spike contribution. MCF7 has two SNPs in the PIK3CA gene, a public SNP: E707K (dbSN#104886003) and the E545K mutation (COSMIC ID# 29328). E545K is a driver mutation and is reported to be heterozygous in MCF7 [15]. Sequencing of pure MCF7 (Table 2) shows a consistent representation of ∼40% - 45%. A549 is reported to be homozygous for both driver mutations KRAS G12S (COSMIC ID #25880) and STK11 Q37* (COSMIC ID #12925) [16, 17]. Our sequencing of pure A549 show consistent representation for both of these SNPs at about 100%.

Surveying all SNP variation by scatter plot analysis shows that two independently derived genomic WBC libraries with R2 = ∼0.9 concordance (Figure 2c). When comparing a WBC library to a 20/40 cell spike the concordance was again R2 = ~0.9. This is to be expected as the minor contribution of the spiked cells is no more than 1%. If the concordance was skewed it could not be due to the spike contribution.

*High read depths yield sensitive and accurate results.* In the reported experiments, the read depths per amplicon were minimally required to be >500 reads. However, for the genes, PIK3CA, KRAS, and STK11 the reads were much higher than >9000 reads per amplicon. This was useful as it proved to enable SNP calls at the 1% frequency. Spikes were constructed where, on average*, five A549 cells were spiked into 400 WBCs. The theoretical allele frequency for the homozygous mutations found in A549 for KRAS G12S and STK11 Q37* using 5 cells /405 cells (or 10 chromosomes /810 chromosomes) is about 1%. These libraries were also constructed with, on average*, 10 MCF7 cells spiked into 400 WBC, thus the allele frequency for the heterozygous PIK3CA mutation E545K was also 1%. In these libraries all A459 and all MCF7 mutations were robustly detected at approximately the expected frequencies (*given Poisson sampling error at this low concentration of cell spike per library [18]). When libraries were constructed with larger numbers (20 A549 /40 MCF7) cells the expected dose response relationship was observed.

In these amplicon libraries not only was there excellent precision and accuracy for the SNP calls but there was a reasonably quantitative relationship between cell spike concentration and library representation. This data suggests that in the future it may be possible to not only detect SNPs from amplicon libraries constructed from CTCs (rare cell isolates) but also show copy number variation if the number of mutation bearing CTCs per library is enumerated.

In summary our experiments show that WGA based Next Generation sequencing libraries are biased with respect to representation. Furthermore we show that these same libraries have many SNP artifacts introduced by the WGA procedure. Thus, analysis WGA-based libraries benefits from parallel library production using multiple different DNA polymerases or the same DNA polymerase multiple times and comparing the amplified nucleic acid sequences to an unamplified, direct sequence libraries for SNP verification and and analysis of CTC (rare cell) based libraries. Direct Sequencing Libraries (DSL) presents an attractive path to developing actionable patient data from Circulating Tumor Cells (CTCs).

### REFERENCES:

1) Ashworth T (1869) A case of cancer in which cells similar to those in the tumors were seen in the blood after death. Australian Med J 14: 146.
2) Allard WJ, Matera J, Miller MC, Repollet M, Connelly MC, et al. (2004) Tumor cells circulate in the peripheral blood of all major carcinomas but not in healthy subjects or patients with nonmalignant diseases. Clin Cancer Res 10: 6897-6904.
3) Momburg F, Moldenhauer G, Hammerling GJ,MollerP (1987) Immunohistochemical study of the expression of a Mr 34,000 human epithelium-specific surface glycoprotein in normal and malignant tissues. Cancer Res 47: 2883-2891.
4) Sequist LV, Bell DW, Lynch TJ, Haber DA (2007) Molecular predictors of response to epidermal growth factor receptor antagonists in non-small-cell lung cancer. J Clin Oncol 25: 587-595.
5) <http://en.wikipedia.org/wiki/C-value>
6) Fuller, C. W., Middendorf, L. R., Benner, S. A., Church, G. M., Harris, T., Huang, X., Jovanovich, S. B., et al. (2009). The challenges of sequencing by synthesis. Nature Biotechnology, 27(11), 1013-1023. doi:10.1038/nbt.1585.
7) Albert, T. J., Molla, M. N., Muzny, D. M., Nazareth, L., Wheeler, D., Song, X., Richmond, T. A., et al. (2007). Direct selection of human genomic loci by microarray hybridization. Nature Methods, 4(11), 903-905. doi:10.1038/nmeth1111.
8) Okou, D. T. D., Steinberg, K. M. K., Middle, C. C., Cutler, D. J. D., Albert, T. J. T., & Zwick, M. E. M. (2007). Microarray-based genomic selection for high-throughput resequencing. Nature Methods, 4(11), 907-909. doi:10.103 8/nmeth1109.
9) Porreca, G. J., Zhang, K., Li, J. B., Xie, B., Austin, D., Vassallo, S. L., LeProust, E. M., et al. (2007). Multiplex amplification of large sets of human exons. Nature Methods, 4(11), 931-936. doi:10.1038/nmeth1110.
10) P. Paterlini-Brechot and N. L. Benali, (2007) "Circulating tumor cells (CTC) detection: clinical impact and future directions," Cancer Letters, vol. 253, no. 2, pp. 180-204.
11) A. G. J. Tibbe, M. C. Miller, and L. W. Terstappen, (2007) "Statistical considerations for enumeration of circulating tumor cells," Cytometry A, vol. 71, no. 3, pp. 154-162, 2007.
12) A. A. Ross, B. W. Cooper, H. M. Lazarus, et al., (1993)"Detection and viability of tumor cells in peripheral blood stem cell collec- tions from breast cancer patients using immunocytochemical and clonogenic assay techniques," Blood, vol. 82, no. 9, pp. 2605-2610.
13) S. Sleijfer, J.-W. Gratama, A. M. Sieuwerts, J. Kraan, J. W. M. Martens, and J. A. Foekens, (2007) "Circulating tumour cell detection on its way to routine diagnostic implementation?" European Journal of Cancer, vol. 43, no. 18, pp. 2645-2650.
14) Allan, A. L., & Keeney, M. (2010). Circulating tumor cell analysis: technical and statistical considerations for application to the clinic. Journal of oncology, 2010, 426218. doi:10.1155/2010/426218
15) <http://on the internet at sanger.ac.uk/perl/genetics/CGP/cosmic?action=sample&id=947352>
16) <http://on the internet at sanger.ac.uk/perl/genetics/CGP/cosmic?action=sample&id=1436014>
17) <http://on the internet at sanger.ac.uk/perl/genetics/CGP/cosmic?action=sample&id=1004698>
18) Taswell, C. (1981). Limiting dilution assays for the determination of immunocompetent cell frequencies. I. Data analysis. Journal of immunology (Baltimore, Md : 1950), 126(4), 1614-1619.

### Example 2

### Dual Enzymatic Amplification to Verify Genomic Mutations in a Rare Cell Population

In order to measure mutations in the DNA genome of CTC's isolated from 2 to 4 ml of whole blood, by any technology, DNA of sufficient quantity and quality is important. Typically, from 2 to 4 ml of whole blood one can expect 2 to 10 CTCs to be recovered. This number of cells must be processed with excellent recovery to ensure that mutation-bearing chromosomes are not lost during processing. Thus, to isolate DNA of sufficient quality and quantity a special approach is required. Conventional methods are not useful as they alter the DNA genomic representation, produce inferior quality DNA and/or result in insufficient quantity from such rare samples for use in a variety of molecular assays such as, but not limited to, QPCR and DNA sequencing.

Isolating DNA from a rare cell population, *e*.*g*., small numbers of blood-derived CTC cells, introduces several obstacles. For example, calibration of sample cell recovery should be enabled with an internal standard. Second, the sample must be transferred, *e*.*g*., from "CHIP" to DNA isolation vessel. Third, sample must be processed, *e.g.,* to isolate DNA. Fourth, DNA must be amplified, *e.g.,* to increase the available DNA.

Internal control spikes are added to samples for performance calibration. Cells are recovered from ISMAC device by centrifugation into 0.5 µL tubes.

Cells are processed for DNA by the following method: Entire cell pellet should be contained within 1 X 500 µL PCR tube. Spin this tube at highest possible RCF for 10 min. VERY GENTLY remove supernatant at first using a pipette but finishing with a microcapillary pipette (Drummond Microcap Cat #1-000-0250).

### Prepare "0.5X" LB2:

50 mM Tris
50 mM HEPES
3 mM SDS
2.5 mM Glycine, pH 8.0

To the LB2 add sufficient Proteinase K (Qiagen Cat #19133) to yield 2 mg/ml final concentration. The final solution of LB2 + Proteinase K is called "Digestion Buffer."

To each tube add 5 µL of this Digestion Buffer. Briefly spin tube, and allow the digestion to proceed as described below:
Incubate at 55°C for 3 Hr
Incubate at 70°C for 1 Hr
Rest at 4°C until ready to proceed or place in -20°C freezer.

DNA is amplified enzymatically.
Starting from a 5 µL genomic DNA (gDNA) preparation
Add 20 µL of sample buffer (GE Healthcare Genome Phi WGA kit)
Run PCR program "WGA" → 3min @ 95°C → 4°C
Add 20 µL reaction buffer+ 2 µL Phi29 enzyme
Step program WGA → 2 hours @ 30°C → 10 min@ 70°C → 4°C.
Add 60 µL Ultra pure H₂O
Spec sample OD 260/280 with Nanodrop

It is understood that the examples and embodiments described herein are for illustrative purposes only and that various modifications or changes in light thereof will be suggested to persons skilled in the art and are to be included within the purview of this application and scope of the appended claims.

## Claims

1. A method for verifying the presence of a genomic mutation in cells of a rare cell population comprising:
a) amplifying and sequencing a portion or the whole genome of the cells of the rare cell population with a first DNA polymerase;
b) amplifying and sequencing a portion or the whole genome of the cells of the rare cell population with a second DNA polymerase, wherein the second DNA polymerase is different from the first DNA polymerase; wherein the amplifying with the first DNA polymerase and second DNA polymerase are performed as parallel amplification reactions in separate reaction mixtures;
c) sequencing without amplifying a portion or the whole genome of a control cell population comprising normal somatic genomic DNA;
d) comparing the genomic sequences obtained in steps a), b) and c), wherein identification of a nucleotide polymorphism that is identical in the genomic sequences obtained in steps a) and b), but different from a nucleotide polymorphism at the same nucleotide position in the genomic sequence obtained in step. c) verify the presence of a genomic mutation in cells of the rare cell population.

2. The method of claim 1, wherein the first DNA polymerase and the second DNA polymerase have different error correction rates.

3. The method of any one of claims 1 to 2 wherein the first DNA polymerase and the second DNA polymerase have different nucleic acid copying fidelities.

4. The method of any one of claims 1 to 3, wherein the first DNA polymerase and/or the second DNA polymerase have 5'→ 3' exonuclease activity.

5. The method of any one of claims 1 to 4, wherein the first DNA polymerase and/or the second DNA polymerase do not have 3'→ 5' exonuclease activity.

6. The method of any one of claims 1 to 5, wherein the first DNA polymerase and/or the second DNA polymerase have helicase and/or strand displacement activity.

7. The method of any one of claims 1 to 6, wherein the first DNA polymerase and the second DNA polymerase are selected from the group consisting of a Φ29 DNA polymerase, a *Thermus aquaticus* (Taq) DNA polymerase, a *Thermus flavus* (Tfl) DNA polymerase, a *Thermus thermophilus* (rTth) DNA polymerase, a *Thermus litoris* (Tli) DNA polymerase, a *Thermotoga maritima* (Tma) DNA polymerase, a *Pyrococcus furiosus* (Pfu) DNA polymerase, a *Bacillus stearothermophilus* (Bst) DNA polymerase, PHUSION® High-Fidelity DNA polymerase, VentR® DNA polymerase, Deep VentR™ DNA polymerase, a Q5™ High-Fidelity DNA polymerase, and REPLI-g DNA polymerase.

8. The method of any one of claims 1 to 7, further comprising the step of isolating the genomic DNA from the cells of a rare cell population.

9. The method of any one of claims 1 to 8, wherein the rare cell population is circulating tumor cells (CTC).

10. The method of claim 9, wherein the CTC are obtained from a blood sample of a subject.

11. The method of any one of claims 9 to 10, wherein the CTC are isolated based on their surface expression of Epithelial cell adhesion molecule (Ep-CAM).

12. The method of any one of claims 1 to 11, wherein the genomic mutation is a single nucleotide polymorphism (SNP).

13. The method of any one of claims 1 to 12, wherein the somatic genomic DNA is from a biological sample selected from the group consisting of white blood cells (WBC), buccal swab, hair bulb or hair follicle.

14. The method of any one of claims 1 to 13, wherein the whole genome of the cells in steps a) and b) is amplified and sequenced.

15. The method of any one of claims 1 to 13, wherein a portion of the whole genome of the cells in steps a) and b) is amplified and sequenced.

16. The method of any one of claims 1 to 15, wherein the portion or the whole genome of the cells is sequenced by performing Next Generation Sequencing.

17. A method for verifying the presence of a genomic mutation in cells of a rare cell population comprising:
a) amplifying a portion or the whole genome of the cells of the rare cell population with a first DNA polymerase;
b) amplifying a portion or the whole genome of the cells of the rare cell population with a second DNA polymerase, wherein the second DNA polymerase is different from the first DNA polymerase; wherein the amplifying with the first DNA polymerase and second DNA polymerase are performed as parallel amplification reactions in separate reaction mixtures;
c) comparing the amplified genomic sequences obtained in steps a) and b) with an unamplified genomic sequence obtained from a control population of cells comprising normal somatic genomic DNA, wherein identification of a nucleotide polymorphism that is identical in the genomic sequences obtained in steps a) and b), but different from a nucleotide polymorphism at the same nucleotide position in the genomic sequence obtained the unamplified genomic sequence verify the presence of a genomic mutation in cells of the rare cell population.

18. The method of claim 17, wherein the amplified and unamplified genomic sequences are compared by one or more procedures comprising sequencing, amplification and/or hybridization.

## Patentansprüche

1. Verfahren zur Bestätigung des Vorliegens einer Genommutation in Zellen einer seltenen Zellpopulation, das Folgendes umfasst:
a) Amplifizieren und Sequenzieren eines Teils oder der Gesamtheit des Genoms der Zellen der seltenen Zellpopulation mit einer ersten DNA-Polymerase;
b) Amplifizieren und Sequenzieren eines Teils oder der Gesamtheit des Genoms der Zellen der seltenen Zellpopulation mit einer zweiten DNA-Polymerase, wobei sich die zweite DNA-Polymerase von der ersten DNA-Polymerase unterscheidet, wobei das Amplifizieren mit der ersten DNA-Polymerase und der zweiten DNA-Polymerase als parallele Ampifikationsreaktionen in getrennten Reaktionsgemischen vorgenommen wird;
c) Sequenzieren eines Teils oder der Gesamtheit des Genoms einer Kontrollzellenpopulation, die normale somatische Genom-DNA aufweist, ohne Amplifikation;
d) Vergleichen der in den Schritten a), b) und c) erhaltenen Genomsequenzen, wobei die Identifikation eines Nucleotidpolynorphismus, der bei den in den Schritten a) und b) erhaltenen Genomsequenzen identisch ist, sich aber von einem Nucleotidpolymorphismus in der gleichen Nucleotidposition in der in dem Schritt c) erhaltenen Genomsequenz unterscheidet, die Anwesenheit einer Genommutation in Zellen der seltenen Zellpopulation bestätigt.

2. Verfahren nach Anspruch 1,
wobei die erste DNA-Polymerase und die zweite DNA-Polymerase unterschiedliche Fehlerkorrektur-Raten aufweisen.

3. Verfahren nach einem der Ansprüche 1 bis 2,
wobei die erste DNA-Polymerase und die zweite DNA-Polymerase eine unterschiedliche Nucleinsäure-Kopierzuverlässigkeit aufweisen.

4. Verfahren nach einem der Ansprüche 1 bis 3,
wobei die erste DNA-Polymerase und/oder die zweite DNA-Polymerase eine 5'→3'-Exonuclease-Aktivität aufweisen.

5. Verfahren nach einem der Ansprüche 1 bis 4,
wobei die erste DNA-Polymerase und/oder die zweite DNA-Polymerase keine 3'→5'-Exonuclease-Aktivität aufweisen.

6. Verfahren nach einem der Ansprüche 1 bis 5,
wobei die erste DNA-Polymerase und/oder die zweite DNA-Polymerase eine Helicase-Aktivität und/oder eine Strangverdrängungsaktivität aufweisen.

7. Verfahren nach einem der Ansprüche 1 bis 6,
wobei die erste DNA-Polymerase und die zweite DNA-Polymerase aus der Gruppe ausgewählt sind, die aus folgenden Komponenten besteht:
einer Φ-29-DNA-Polymerase, einer *Thermus aquaticus* (TaQ)-DNA-Polymerase, einer *Thermus flavus* (Tfl)-DNA-Polymerase, einer *Thermus thermophilus* (rTth)-DNA-Polymerase, einer *Thermus litoris* (Tli)-DNA-Polymerase, einer *Thermotoga maritima* (Tma)-DNA-Polymerase, einer *Pyrococcus furiosus* (Pfu)-DNA-Polymerase, einer *Bacillus stearothermophilus* (Bst)-DNA-Polymerase, PHUSION^{R}-High-Fidelity-DNA-Polymerase, VentR^{R}-DNA-Polymerase, Deep VentR^{R}-DNA-Polymerase, einer Q5^{R}-High Fidelity-DNA-Polymerase und REPLI-g-DNA-Polymerase.

8. Verfahren nach einem der Ansprüche 1 bis 7,
das ferner den Schritt des Isolierens der Genom-DNA aus den Zellen der seltenen Zellpopulation umfasst.

9. Verfahren nach einem der Ansprüche 1 bis 8,
wobei es sich bei den Zellen der seltenen Zellpopulation um zirkulierende Tumorzellen (CTC) handelt.

10. Verfahren nach Anspruch 9,
wobei die CTC aus einer Blutprobe eines Subjekts gewonnen werden.

11. Verfahren nach einem der Ansprüche 9 bis 10,
wobei die CTC auf der Basis ihrer Oberflächenexpression des Epithelzellen-Haftmoleküls (Ep-CAM) isoliert werden.

12. Verfahren nach einem der Ansprüche 1 bis 11,
wobei es sich bei der Genommutation um einen einzelnen Nucleotidpolymorphismus (SNP) handelt.

13. Verfahren nach einem der Ansprüche 1 bis 12,
wobei die somatische Genom-DNA aus einer biologischen Probe stammt, die aus der Gruppe ausgewählt ist, die besteht aus weißen Blutkörperchen (WBC), einem bukkalen Abstrich, einem Haarbulbus oder einem Haarfollikel.

14. Verfahren nach einem der Ansprüche 1 bis 13,
wobei in den Schritten a) und b) das gesamte Genom der Zellen amplifiziert und sequenziert wird.

15. Verfahren nach einem der Ansprüche 1 bis 13,
wobei in den Schritten a) und b) ein Teil des gesamten Genoms der Zellen amplifiziert und sequenziert wird.

16. Verfahren nach einem der Ansprüche 1 bis 15,
wobei ein Teil oder die Gesamtheit des Genoms der Zellen durch Vornahme einer Sequenzierung der nächsten Generation (Next Generation Sequencing) sequenziert wird.

17. Verfahren zur Bestätigung des Vorliegens einer Genommutation in Zellen einer seltenen Zellpopulation, das Folgendes umfasst:
a) Amplifizieren eines Teils oder der Gesamtheit des Genoms der Zellen der seltenen Zellpopulation mit einer ersten DNA-Polymerase;
b) Amplifizieren eines Teils oder der Gesamtheit des Genoms der Zellen der seltenen Zellpopulation mit einer zweiten DNA-Polymerase, wobei sich die zweite DNA-Polymerase von der ersten DNA-Polymerase unterscheidet, wobei das Amplifizieren mit der ersten DNA-Polymerase und der zweiten DNA-Polymerase als parallele Ampifikationsreaktionen in getrennten Reaktionsgemischen vorgenommen wird;
c) Vergleichen der in den Schritten a) und b) erhaltenen amplifizierten Genomsequenzen mit einer nicht-amplifizierten Genomsequenz, die aus einer Kontrollpopulation von Zellen, die normale somatische Genom-DNA umfassen, erhalten worden ist, wobei die Identifikation eines Nucleotidpolymorphismus, der bei den in den Schritten a) und b) erhaltenen Genomsequenzen identisch ist, sich aber von einem Nucleotidpolymorphismus in der gleichen Nucleotidposition der erhaltenen Genomsequenz der nicht-amplifizierten Genomsequenz unterscheidet, die Anwesenheit einer Mutation in Zellen der seltenen Zellpopulation bestätigt.

18. Verfahren nach Anspruch 17,
wobei die amplifizierten und nicht-amplifizierten Genomsequenzen durch ein oder mehr Verfahren, die eine Sequenzierung, Amplifikation und/oder Hybridiserung umfassen, verglichen werden.

## Revendications

1. Procédé de vérification de la présence d'une mutation du génome dans des cellules d'une population de cellules rares comprenant :
(a) l'amplification et le séquençage d'une partie ou de l'ensemble du génome des cellules de la population de cellules rares avec une première ADN polymérase ;
(b) l'amplification et le séquençage d'une partie ou de l'ensemble du génome des cellules de la population de cellules rares avec une deuxième ADN polymérase, selon lequel le deuxième ADN polymérase est différente de la première ADN polymérase ; selon lequel l'amplification avec la première ADN polymérase et la deuxième ADN polymérase s'effectue sous forme de réactions d'amplification parallèles dans des mélanges réactionnels séparés ;
(c) le séquençage sans amplification d'une partie ou de l'ensemble du génome d'une population de cellules témoins comprenant de l'ADN génomique somatique normal ;
(d) la comparaison des séquences génomiques obtenues lors des étapes (a), (b), et (c), selon laquelle l'identification d'un polymorphisme nucléotidique qui est identique dans les séquences génomiques obtenues lors des étapes (a) et (b), mais différent d'un polymorphisme nucléotidique à la même position nucléotidique dans la séquence génomique obtenue lors de l'étape (c) afin de confirmer la présence d'une mutation génomique dans des cellules de la population de cellules rares.

2. Procédé selon la revendication 1, selon lequel la première ADN polymérase et la deuxième ADN polymérase présentent des taux de correction d'erreurs différents.

3. Procédé selon l'une quelconque des revendications 1 ou 2, selon lequel la première ADN polymérase et la deuxième ADN polymérase présentent des fidélités de copie d'acide nucléique différentes.

4. Procédé selon l'une quelconque des revendications 1 à 3, selon lequel la première ADN polymérase et/ou la deuxième ADN polymérase présentent une activité exonucléase 5'→3'.

5. Procédé selon l'une quelconque des revendications 1 à 4, selon lequel la première ADN polymérase et/ou la deuxième ADN polymérase présentent une activité exonucléase 3'→5'.

6. Procédé selon l'une quelconque des revendications 1 à 5, selon lequel la première ADN polymérase et/ou la deuxième ADN polymérase présentent une activité hélicase et/ou de déplacement de brin.

7. Procédé selon l'une quelconque des revendications 1 à 6, selon lequel la première ADN polymérase et la deuxième ADN polymérase sont choisies dans le groupe consistant en une ADN polymérase φ29, une ADN polymérase *Thermus aquaticus* (Taq), une ADN polymérase *Thermus flavus* (Tfl), une ADN polymérase *Thermus thermophilus* (rTth), une ADN polymérase *Thermus litoris* (Tli), une ADN polymérase *Thermotoga maritima* (Tma), une ADN polymérase *Pyrococcus furiosus* (Pfu), une ADN polymérase *Bacillus stearothermophilus* (Bst), l'ADN polymérase PHUSION® haute-fidélité, l'ADN polymérase VentR®, l'ADN polymérase Deep VentR™, une ADN polymérase Q5™ haute-fidélité, une ADN polymérase REPLI-g.

8. Procédé selon l'une quelconque des revendications 1 à 7, comprenant en outre le fait d'isoler l'ADN génomique des cellules de la population de cellules rares.

9. Procédé selon l'une quelconque des revendications 1 à 8, selon lequel la population de cellules rares est une population de cellules tumorales circulantes (CTC).

10. Procédé selon la revendication 9, selon lequel les CTC sont obtenues à partir d'un échantillon de sang d'un sujet.

11. Procédé selon l'une quelconque des revendications 9 ou 10, selon lequel les CTC sont isolées à partir de leur expression surfacique de la molécule d'adhérence à la cellule épithéliale (Ep-CAM).

12. Procédé selon l'une quelconque des revendications 1 à 11, selon lequel la mutation génomique est un polymorphisme à nucléotide unique (SNP).

13. Procédé selon l'une quelconque des revendications 1 à 12, selon lequel l'ADN génomique somatique est obtenu à partir d'un échantillon biologique choisi dans le groupe consistant en les globules blancs sanguins (WBC), un frottis buccal, un bulbe pilaire, ou un follicule pileux.

14. Procédé selon l'une quelconque des revendications 1 à 13, selon lequel l'ensemble du génome des cellules des étapes (a) et (b) est amplifié et séquencé.

15. Procédé selon l'une quelconque des revendications 1 à 13, selon lequel une partie de l'ensemble du génome des cellules des étapes (a) et (b) est amplifiée et séquencée.

16. Procédé selon l'une quelconque des revendications 1 à 15, selon lequel la partie ou l'ensemble du génome des cellules est séquencé(e) par la technique de séquençage nouvelle génération (NGS).

17. Procédé de confirmation de la présence d'une mutation génomique dans des cellules d'une population de cellules rares, comprenant :
(a) l'amplification d'une partie ou de l'ensemble du génome des cellules de la population de cellules rares avec une première ADN polymérase ;
(b) l'amplification d'une partie ou de l'ensemble du génome des cellules de la population de cellules rares avec une deuxième ADN polymérase, selon lequel le deuxième ADN polymérase est différente de la première ADN polymérase ; selon lequel l'amplification avec la première ADN polymérase et la deuxième ADN polymérase s'effectue sous forme de réactions d'amplification parallèles dans des mélanges réactionnels séparés ;
(c) la comparaison des séquences génomiques amplifiées obtenues lors des étapes (a) et (b) avec une séquence génomique non-amplifiée obtenue à partir d'une population de cellules témoins comprenant de l'ADN génomique somatique normal, selon laquelle l'identification d'un polymorphisme nucléotidique qui est identique dans les séquences génomiques obtenues lors des étapes (a) et (b), mais différent d'un polymorphisme nucléotidique à la même position nucléotidique dans la séquence génomique obtenue à partir d'une séquence non-amplifiée, afin de confirmer la présence d'une mutation génomique dans des cellules de la population de cellules rares.

18. Procédé selon la revendication 17, selon lequel les séquences génomiques amplifiées et non-amplifiées sont comparées selon une ou plusieurs procédures comprenant le séquençage, l'amplification et/ou l'hybridation.
